# EUROPEAN PATENT SPECIFICATION

(11) **EP 3 086 763 B1**
(45) Date of publication and mention of the grant of the patent: **03.07.2019**
(21) Application number: 14824431.2
(22) Date of filing: 17.12.2014
(51) Int. Cl.: A61K 8/34, A61K 8/37, A61K 8/60, A61K 8/06, A61Q 1/10, A61Q 1/14, A61Q 3/00, A61Q 5/00, A61Q 5/02, A61Q 19/00, A61Q 19/10

(54) **COSMETIC COMPOSITION COMPRISING AN OIL, A NONIONIC SURFACTANT AND A C-GLYCOSIDE COMPOUND**
KOSMETISCHE ZUSAMMENSETZUNG MIT EINEM ÖL, EINEM NICHTIONISCHEN TENSID UND EINER C-GLYKOSIDVERBINDUNG
COMPOSITION COSMÉTIQUE COMPRENANT UNE HUILE, UN TENSIOACTIF NON IONIQUE ET UN COMPOSÉ C-GLYCOSIDIQUE

(30) Priority: 24.12.2013 FR 1363528
(43) Date of publication of application: 02.11.2016
(73) Proprietor: L'Oréal, 75008 Paris (FR)
(72) Inventor: DERRIPS, Céline, 91380 Chilly Mazarin (FR); JOUY, Chantal, F-75009 Paris (FR); BERNARD, Anne-Laure, Clark, NJ 07066 (US)
(74) Representative: L'Oreal
(86) International application number: PCT/EP2014/078160
(87) International publication number: WO 2015/097029

(56) References cited:
- EP-A1- 1 172 077
- WO-A1-2010/067036
- DE-A1-102011 015 191
- FR-A1- 2 903 002
- US-A1- 2008 008 673

## Description

The present invention relates to a composition, especially a cosmetic composition, in the form of a nanoemulsion or a microemulsion as described according to the claims.

Oil-in-water (O/W) emulsions or water-in-oil (W/O) emulsions are known in the field of cosmetics and dermatology, in particular for preparing cosmetic products, such as milks, creams, toners, serums or eaux de toilette. In particular, a fine emulsion such as an O/W nanoemulsion or microemulsion is particularly advantageous in cosmetic products on account of its transparent or slightly translucent appearance.

It is known practice in the field of cosmetics or dermatology to use oil-in-water (O/W) emulsions. These emulsions, which consist of an oily phase (or a lipophilic phase) dispersed in an aqueous phase, have an outer aqueous phase and are therefore products that are more pleasant to use on account of the fresh sensation they afford. However, they have the drawback of lacking stability when the amount of oil present is too high. At the present time, for certain applications, it is advantageous to have a large amount of oils, given that oils give the skin a comfortable feel, nourish it and may also remove makeup therefrom when these oils have makeup-removing properties. Furthermore, it is advantageous to have fine emulsions, i.e. emulsions in which the oily phase is in the form of very small droplets, i.e. droplets less than 4 µm in size, given that these fine emulsions afford a pleasant cosmetic sensation and are generally more stable than coarse emulsions. The patent documents US 2008/0008673 and WO 2010/067036 both concern cosmetic compositions comprising a C-glycoside derivative.

These emulsions may be prepared, in particular, via the phase inversion temperature technique (PIT emulsions), in which the mean size of the globules constituting the oily phase is within given limits, namely between 0.1 and 4 µm (100 to 4000 nm). The principle of phase inversion temperature (PIT) emulsification is, in theoretical terms, known to those skilled in the art; it was described in 1968 by K. Shinoda (J. Chem. Soc. Jpn., 1968, 89, 435). It has been shown that this emulsification technique allows stable, fine emulsions to be obtained (K. Shinoda and H. Saito, J. Colloid Interface Sci., 1969, 30, 258). This technique was applied in cosmetics as early as 1972 by Mitsui et al. ("Application of the phase-inversion-temperature method to the emulsification of cosmetics"; T. Mitsui, Y. Machida and F. Harusawa, American Cosmet. Perfum., 1972, 87, 33).

The principle of this technique is as follows: an O/W emulsion (introduction of the aqueous phase into the oily phase) is prepared at a temperature which should be above the phase inversion temperature of the system, which is the temperature at which the equilibrium between the hydrophilic and lipophilic properties of the emulsifier(s) used is reached; at a higher temperature, i.e. above the phase inversion temperature (>PIT), the emulsion is of water-in-oil type, and, when it cools down, this emulsion inverts at the phase inversion temperature, to become an emulsion of oil-in-water type, passing beforehand through a microemulsion state. This process makes it readily possible to obtain emulsions with a diameter generally less than 4 µm. The emulsifying surfactants of oil-in-water type conventionally used have an HLB (hydrophilic-lipophilic balance) in the range from 8 to 18. On account of their amphiphilic structure, these emulsifiers are located at the oily phase/aqueous phase interface, and thus stabilize the dispersed oil droplets.

However, it is difficult to produce fine O/W emulsions containing a large amount of oily phase, given that such emulsions have a tendency to become destabilized, this destabilization leading to coalescence and separation of the aqueous and oily phases with release of oil. In order to improve the stability of these emulsions, the concentration of emulsifiers may be increased; however, a high concentration of emulsifiers may lead to a rough, adhesive or tacky feel and to safety concerns with respect to the skin, the eyes and the scalp.

In particular, a fine emulsion such as an O/W nanoemulsion or microemulsion is particularly advantageous in cosmetic products on account of its transparent or slightly translucent appearance.

For example, JP-A-H09-110635 describes a fine emulsion which is formed by using a combination of polyglyceryl fatty acid ester as surfactant and a C₁₀-C₂₂ fatty 2-hydroxy acid. Furthermore, JP-A-H11-71256 describes a fine emulsion which is formed by using a combination of polyglyceryl fatty acid ester and betaine.

However, when certain types of nonionic surfactant are used for preparing fine emulsions, the transparent or slightly translucent appearance of the emulsion and the stability of the emulsion are insufficient.

One object of the present invention is to propose a cosmetic composition in the form of a nanoemulsion or microemulsion that has a transparent or slightly translucent, preferably transparent, appearance.

After detailed investigations, the inventors have discovered that it is possible to produce a stable cosmetic composition in the form of a nanoemulsion or microemulsion that has a transparent or slightly translucent, preferably transparent, appearance of the emulsion, even when a nonionic surfactant was used that made it difficult to form a fine emulsion such as a nanoemulsion or microemulsion.

Consequently, the present invention relates to a cosmetic composition in the form of a nanoemulsion or microemulsion, according to claim 1.

Given that the cosmetic composition according to the present invention may have a transparent or slightly translucent appearance, the composition may preferably be used for lotions and the like. Furthermore, the cosmetic composition according to the present invention may produce a pleasant texture and afford moisturizing properties and also increased suppleness. Furthermore, if the dispersed phase is an oily phase and comprises one or more lipophilic or even amphiphilic active components, the oily dispersed phase may function as a vehicle for the active substance and accelerate the penetration of the active components into the skin, or may distribute the active components on the skin.

Furthermore, the present invention also relates to a non-therapeutic process for treating the skin, the hair, mucous membranes, the nails, the eyelashes, the eyelids and/or the scalp, characterized in that the cosmetic composition according to the present invention is applied to the skin, the hair, mucous membranes, the nails, the eyelashes, the eyelids or the scalp.

Furthermore, the present invention also relates to a use of the cosmetic composition according to the present invention as or in care products and/or washing products and/or makeup products and/or makeup-removing products for bodily and/or facial skin and/or mucous membranes and/or the scalp and/or the hair and/or the nails and/or the eyelashes and/or the eyelids.

The cosmetic composition according to the present invention is described in greater detail hereinbelow.

The cosmetic composition according to the present invention comprises at least one oil. According to the present invention, the term "oil" denotes a fatty compound or substance that is in the form of a liquid at room temperature (25°C) and at atmospheric pressure (760 mmHg). As oils, those generally used in cosmetics may be used alone or in combinations thereof. These oils may be volatile or non-volatile, preferably non-volatile.

The oil may be a non-polar oil such as a hydrocarbon oil, a silicone oil or the like; a polar oil such as a plant or animal oil and an ester oil or an ether oil; or a mixture thereof.

It is preferable for the oil (a) to be chosen from the group consisting of oils of plant origin, animal oils, synthetic oils, silicone oils and hydrocarbon oils.

As examples of plant oils, mention may be made, for example, of linseed oil, camellia oil, macadamia oil, corn oil, castor oil, olive oil, avocado oil, sasanqua oil, safflower oil, jojoba oil, sunflower oil, almond oil, rapeseed oil, sesame oil, soybean oil, groundnut oil, argan oil and apricot kernel oil, and mixtures thereof.

As examples of animal oils, mention may be made, for example, of squalene and squalane.

As examples of synthetic oils, mention may be made of alkanes such as isododecane and isohexadecane, fatty esters, fatty ethers and artificial C6-C22 acid triglycerides.

The fatty esters are preferably liquid esters of linear or branched, saturated or unsaturated C₁-C₂₆ aliphatic monoacids or polyacids and of linear or branched, saturated or unsaturated C₁-C₂₆ aliphatic monoalcohols or polyalcohols, the total number of carbon atoms in the fatty esters being greater than or equal to 10.

Preferably, for the monoalcohol esters, at least one from among the alcohol and the acid is branched.

Among the monoesters of monoacids and of monoalcohols, mention may be made of ethyl palmitate, ethylhexyl palmitate, isopropyl palmitate, dicaprylyl carbonate, alkyl myristates such as isopropyl myristate or ethyl myristate, isocetyl stearate, 2-ethylhexyl isononanoate, isononyl isononanoate, isodecyl neopentanoate and isostearyl neopentanoate.

Esters of C₄-C₂₂ dicarboxylic or tricarboxylic acids and of C₁-C₂₂ alcohols and esters of monocarboxylic, dicarboxylic or tricarboxylic acids and of non-saccharide C₄-C₂₆ dihydroxy, trihydroxy, tetrahydroxy or pentahydroxy alcohols may also be used.

Mention may in particular be made of: diethyl sebacate; isopropyllauryl sarcosinate; diisopropyl sebacate; bis(2-ethylhexyl) sebacate; diisopropyl adipate; di-n-propyl adipate; dioctyl adipate; bis(2-ethylhexyl) adipate; diisostearyl adipate; bis(2-ethylhexyl) maleate; triisopropyl citrate; triisocetyl citrate; triisostearyl citrate; glyceryl trilactate; glyceryl trioctanoate; trioctyldodecyl citrate; trioleyl citrate; neopentyl glycol diheptanoate; and diethylene glycol diisononanoate.

Fatty esters that may be used include sugar esters and diesters of C₆-C₃₀ and preferably C₁₂-C₂₂ fatty acids. It is recalled that the term "sugar" means hydrocarbon-based compounds comprising oxygen containing several alcohol functions, with or without aldehyde or ketone functions, and which comprise at least 4 carbon atoms. These sugars may be monosaccharides, oligosaccharides or polysaccharides.

Examples of suitable sugars that may be mentioned include saccharose (or sucrose), glucose, galactose, ribose, fucose, maltose, fructose, mannose, arabinose, xylose and lactose, and derivatives thereof, in particular alkyl derivatives such as methyl derivatives, for example methylglucose.

The sugar esters of fatty acids may be chosen especially from the group comprising the esters or mixtures of esters of sugars described previously and of linear or branched, saturated or unsaturated C₆-C₃₀ and preferably C₁₂-C₂₂ fatty acids. If they are unsaturated, these compounds may contain from one to three conjugated or unconjugated double bonds.

The esters according to this variant may also be chosen from monoesters, diesters, triesters, tetraesters and polyesters, and mixtures thereof.

These esters may be, for example, oleates, laurates, palmitates, myristates, behenates, cocoates, stearates, linoleates, linolenates, caprates and arachidonates, or mixtures thereof such as, in particular, oleopalmitate, oleostearate and palmitostearate mixed esters, and also pentaerythrityl tetraethylhexanoate.

More particularly, use is made of monoesters and diesters and in particular sucrose, glucose or methylglucose monooleates or dioleates, stearates, behenates, oleopalmitates, linoleates, linolenates and oleostearates.

An example that may be mentioned is the product sold under the name Glucate® DO by the company Amerchol, which is a methylglucose dioleate.

As preferred examples of fatty esters, mention may be made, for example, of diisopropyl adipate, dioctyl adipate, 2-ethylhexyl hexanoate, ethyl laurate, cetyl octanoate, octyldodecyl octanoate, isodecyl neopentanoate, myristyl propionate, 2-ethylhexyl 2-ethylhexanoate, 2-ethylhexyl octanoate, 2-ethylhexyl caprylate/caprate, methyl palmitate, ethyl palmitate, isopropyl palmitate, ethylhexyl palmitate, isohexyl laurate, hexyl laurate, isocetyl stearate, isopropyl isostearate, isopropyl myristate, isodecyl oleate, glyceryl tris(2-ethylhexanoate), pentaerythrityl tetrakis(2-ethylhexanoate), 2-ethylhexyl succinate and diethyl sebacate, and mixtures thereof.

As examples of artificial triglycerides, mention may be made, for example, of glyceryl trimyristate, glyceryl tripalmitate, glyceryl trilinolenate, glyceryl trilaurate, glyceryl tricaprate, glyceryl tricaprylate, glyceryl tri(caprate/caprylate) and glyceryl tri(caprate/caprylate/linolenate).

As examples of silicone oils, mention may be made, for example, of linear organopolysiloxanes such as dimethylpolysiloxane, methylphenylpolysiloxane, methylhydrogenopolysiloxane and the like; cyclic organopolysiloxanes such as octamethylcyclotetrasiloxane, decamethylcyclopentasiloxane, dodecamethylcyclohexasiloxane and the like; and mixtures thereof.

Preferably, the silicone oil is chosen from liquid polydialkylsiloxanes, in particular liquid polydimethylsiloxanes (PDMS) and liquid polyorganosiloxanes comprising at least one aryl group.

These silicone oils may also be organomodified. The organomodified silicones that may be used according to the present invention are silicone oils as defined above comprising in their structure one or more organofunctional groups linked via a hydrocarbon-based group.

Organopolysiloxanes are defined in greater detail in Walter Noll's Chemistry and Technology of Silicones (1968), Academic Press. They may be volatile or non-volatile.

Volatile or non-volatile silicone oils, such as volatile or non-volatile polydimethylsiloxanes (PDMS) containing a linear or cyclic silicone chain, which are liquid or pasty at room temperature, in particular cyclopolydimethylsiloxanes (cyclomethicones) such as cyclohexasiloxane; polydimethylsiloxanes containing alkyl, alkoxy or phenyl groups that are pendent or at the end of the silicone chain, the said groups containing from 2 to 24 carbon atoms; phenyl silicones such as phenyl trimethicones, phenyl dimethicones, phenyltrimethylsiloxydiphenylsiloxanes, diphenyl dimethicones, diphenylmethyldiphenyltrisiloxanes, 2-phenyl ethyl trimethylsiloxysilicates and polymethylphenylsiloxanes, may be used.

The hydrocarbon-based oils may be chosen from:
- linear or branched, optionally cyclic, C₆-C₁₆ lower alkanes. Examples that may be mentioned include hexane, undecane, dodecane, tridecane, and isoparaffins, for instance isohexadecane, isododecane and isodecane; and
- linear or branched hydrocarbons containing more than 16 carbon atoms, such as liquid paraffins, a liquid paraffin gel, polydecenes and hydrogenated polyisobutenes such as Parleam®, and squalane.

As preferred examples of hydrocarbon-based oils, mention may be made, for example, of linear or branched hydrocarbons such as mineral oil (for example liquid paraffin), paraffin, petroleum jelly or petrolatum, naphthalens and the like; hydrogenated polyisobutene, isoeicosane, and a decene/butene copolymer; and mixtures thereof.

It is also preferable for the oil (a) to be chosen from oils with a molecular weight of less than 600 g/mol.

Preferably, the oil (a) is chosen from fatty esters containing one or more C₁-C₁₂ hydrocarbon-based chains (for example isopropyl myristate, isopropyl palmitate, isononyl isononanoate and ethylhexyl palmitate), hydrocarbon oils (for example isododecane, isohexadecane and squalane), oils of branched and/or unsaturated C₁₂-C₃₀ fatty alcohol type such as octyldodecanol or oleyl alcohol, and fatty ethers such as dicaprylyl ether.

The amount of the oil (a) in the cosmetic composition according to the present invention may be in the range from 0.1% to 50% by weight, preferably from 1% to 40% by weight and more preferably from 5% to 30% by weight relative to the total weight of the composition.

### [Nonionic surfactant]

The cosmetic composition according to the present invention may comprise at least one specific nonionic surfactant.

The nonionic surfactant has an HLB (hydrophilic-lipophilic balance) value of from 8.0 to 14.0, preferably from 9.0 to 13.5 and more preferably from 10.0 to 13.0. If two or more nonionic surfactants are used, the HLB value is determined by the mean weight of the HLB values of all the nonionic surfactants.

The term "HLB" is well known to those skilled in the art, and denotes the hydrophilic-lipophilic balance of a surfactant.

The HLB or hydrophilic-lipophilic balance of the surfactant(s) used according to the invention is the HLB according to Griffin, defined in the publication J. Soc. Cosm. Chem. 1954 (Vol. 5), pages 249-256 or the HLB determined experimentally and as described in the publication from the authors F. Puisieux and M. Seiller, entitled Galenica 5: Les systemes disperses - Tome I - Agents de surface et emulsions [Galenica 5: Dispersed systems - Volume I - Surface agents and emulsions] - Chapter IV - Notions de HLB et de HLB critique [Notions of HLB and of critical HLB], pages 153-194 - paragraph 1.1.2. Determination de HLB par voie experimentale [Experimental determination of HLB], pages 164-180.

It is preferably the calculated HLB values that should be taken into account.

The calculated HLB is defined as being the following coefficient:
calculated HLB = 20 × molar mass of the hydrophilic part / total molar mass.

For an oxyethylenated fatty alcohol, the hydrophilic part corresponds to the oxyethylene units fused to the fatty alcohol and the calculated HLB value then corresponds to the HLB value according to Griffin (Griffin W.C., J. Soc. Cosmet. Chemists, 5, 249,1954).

The nonionic surfactant with an HLB value of from 8.0 to 14.0, preferably from 9.0 to 13.5 and more preferably from 10.0 to 13.0 may be chosen from:
(1) surfactants that are fluid at a temperature of less than or equal to 45°C, chosen from polyglycerols comprising from 2 to 12 glycerol units, and of at least one fatty acid comprising at least one saturated or unsaturated, linear or branched C₈-C₂₂ alkyl chain,
(2) mixed esters of fatty acid or of fatty alcohol, of carboxylic acid and of glycerol,

The surfactants (1) that are fluid at a temperature of less than or equal to 45°C may be, in particular:
- glyceryl laurate comprising 2 glycerol units, sold by the company Solvay;

The mixed esters of fatty acid or of fatty alcohol (2), of carboxylic acid and of glycerol, which may be used as nonionic surfactant above, may be chosen in particular from the group comprising mixed esters of fatty acid or of fatty alcohol with an alkyl chain containing from 8 to 22 carbon atoms, and of α-hydroxy acid and/or of succinic acid, with glycerol. The α-hydroxy acid may be, for example, citric acid, lactic acid, glycolic acid or malic acid, and mixtures thereof.

The alkyl chain of the fatty acids or fatty alcohols from which are derived the mixed esters that may be used in the emulsion of the invention may be linear or branched, and saturated or unsaturated. They may in particular be stearate, isostearate, linoleate, oleate, behenate, arachidonate, palmitate, myristate, laurate, caprate, isostearyl, stearyl, linoleyl, oleyl, behenyl, myristyl, lauryl or capryl chains, and mixtures thereof.

As examples of mixed esters that may be used in the emulsion of the invention, mention may be made of the mixed ester of glycerol and the mixture of citric acid, lactic acid, linoleic acid and oleic acid (CTFA name: glyceryl citrate/lactate/linoleate/oleate) sold by the company Hüls under the name Imwitor 375; the mixed ester of succinic acid and of isostearyl alcohol with glycerol (CTFA name: isostearyl-diglyceryl succinate) sold by the company Hüls under the name Imwitor 780 K; the mixed ester of citric acid and of stearic acid with glycerol (CTFA name: glyceryl stearate-citrate) sold by the company Hüls under the name Imwitor 370; the mixed ester of lactic acid and of stearic acid with glycerol (CTFA name: glyceryl stearate-lactate) sold by the company Danisco under the name Lactodan B30 or Rylo LA30.

It is preferable for the nonionic surfactant (b) with an HLB value of from 8.0 to 14.0, preferably from 9.0 to 13.5 and more preferably from 10.0 to 13.0 to be chosen from:
- polyglyceryl monolaurate or dilaurate comprising 3 to 6 glycerol units,
- polyglyceryl mono(iso)stearate comprising 3 to 6 glycerol units,
- polyglyceryl monooleate comprising 3 to 6 glycerol units, and
- polyglyceryl dioleate comprising 3 to 6 glycerol units.

According to a preferred embodiment of the present invention, the nonionic surfactant with an HLB value from 8.0 to 14.0, preferably from 9.0 to 13.5 and more preferably from 10.0 to 13.0 is chosen from polyglyceryl fatty acid esters and monooxyethylene or polyoxyethylene fatty acid esters.

It is preferable for the polyglyceryl fatty acid ester to comprise esters of a fatty acid and of polyglycerol containing 70% or more of polyglycerol in which the degree of polymerization is 4 or more, preferably esters of a fatty acid and of polyglycerol containing an amount greater than or equal to 60% of polyglycerol in which the degree of polymerization is between 4 and 11, and more preferably esters of a fatty acid and of polyglycerol containing an amount greater than or equal to 30% of polyglycerol in which the degree of polymerization is 5.

The polyglyceryl fatty acid ester may be chosen from monoesters, diesters and triesters of a saturated or unsaturated acid, preferably a saturated acid, comprising 2 to 30 carbon atoms, preferably 6 to 30 carbon atoms and more preferably 8 to 30 carbon atoms, such as lauric acid, oleic acid, stearic acid, isostearic acid, capric acid, caprylic acid and myristic acid.

It is preferable for the polyglyceryl fatty acid ester to be chosen from the group consisting of PG-4 laurate, PG-5 laurate, PG-5 dilaurate, PG-5 oleate, PG-5 dioleate, PG-6 tricaprylate, PG-5 myristate, PG-5 trimyristate, PG-5 stearate, PG-5 isostearate, PG-5 trioleate, PG-6 caprylate and PG-6 tricaprylate.

It is preferable for the monooxyethylene or polyoxyethylene fatty acid ester to contain a (poly)oxyalkylene fragment derived from 1 to 20 oxyalkylenes, preferably from 3 to 15 oxyalkylenes and more preferably from 8 to 10 oxyalkylenes.

The oxyalkylene fragment may be derived from alkylene glycols such as ethylene glycol, propylene glycol, butylene glycol, pentylene glycol, hexylene glycol, and the like. The oxyalkylene fragment may contain a number of moles of ethylene oxide and/or propylene oxide of between 1 and 100 and preferably between 2 and 50. Advantageously, the nonionic surfactants do not comprise any oxypropylene units.

The monooxyethylene or polyoxyethylene fatty acid ester may be chosen from monoesters and diesters of a saturated or unsaturated acid, preferably a saturated acid, comprising from 2 to 30 carbon atoms, preferably from 6 to 30 carbon atoms and more preferably from 8 to 30 carbon atoms, such as lauric acid, oleic acid, stearic acid, isostearic acid, capric acid, caprylic acid and myristic acid.

Examples of monooxyethylene or polyoxyethylene fatty acid esters that may be mentioned comprise a linear or branched, saturated or unsaturated C₂-C₃₀, preferably C₆-C₃₀ and more preferably C₈-C₂₂ acid esters of polyethylene glycols.

Examples of monooxyethylene or polyoxyethylene fatty acid esters that may be mentioned comprise adducts of ethylene oxide with lauric acid, myristic acid, palmitic acid, stearic acid, isostearic acid, oleic acid or behenic acid esters, and mixtures thereof, in particular those containing from 8 to 30 oxyethylene groups, such as PEG-8 to PEG-30 laurate (under the CTFA names: PEG-8 laurate to PEG-30 laurate); PEG-8 to PEG-30 myristate (under the CTFA names: PEG-8 myristate to PEG-30 myristate); PEG-8 to PEG-30 palmitate (under the CTFA names: PEG-8 palmitate to PEG-30 palmitate); PEG-8 to PEG-30 stearate (under the CTFA names: PEG-8 stearate to PEG-30 stearate); PEG-8 to PEG-30 isostearate (under the CTFA names: PEG-8 isostearate to PEG-30 isostearate); PEG-8 to PEG-30 oleate (under the CTFA names: PEG-8 oleate to PEG-30 oleate); PEG-8 to PEG-30 behenate (under the CTFA names: PEG-8 behenate to PEG-30 behenate); and mixtures thereof.

It is preferable for the fatty acid ester of polyglycol to be chosen from the group consisting of PEG-8 isostearate, PEG-8 stearate, PEG-10 isostearate, PEG-10 oleate, PEG-10 isocetyl ether, PEG-10 behenyl ether and PEG-10 isostearyl ether, and a mixture thereof.

The amount of the nonionic surfactant (b) with an HLB value of from 8.0 to 14.0, preferably from 9.0 to 13.5 and more preferably from 10.0 to 13.0 in the cosmetic composition according to the present invention is not limited, and may be in the range from 0.1 % to 30% by weight, preferably from 1% to 25% by weight and more preferably from 3% to 20% by weight relative to the total weight of the composition.

### [C-glycoside compound]

The cosmetic composition according to the present invention comprises at least one C-glycoside compound.

The C-glycosides are preferably of general formula (I) below: 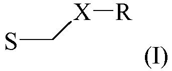 in which:
- R represents a saturated or unsaturated C₁-C₁₀ and in particular C₁-C₄ alkyl radical, which may be optionally substituted with at least one radical chosen from OH, COOH, Y and COOR"₂ with R"₂ being a saturated C₁-C₄ alkyl radical, Y denotes a phenyl radical or a heterocycle, optionally substituted with 1 to 5 (ORₐ) groups,
- S represents a monosaccharide or a polysaccharide comprising up to 20 sugar units, in particular up to 6 sugar units, in pyranose and/or furanose form and of the L and/or D series, it being possible for said monosaccharide or polysaccharide to be substituted with a hydroxyl group which must be free, and optionally with one or more optionally protected amine functions, and
- X represents a radical chosen from the following groups: -CO-, -CH(OR')-, -CH (NH₂)-, CHNR_{b}R_{c}; CHNHOR_{d}, -C(OR')-, -C (NH₂)-, CNR_{b}R_{c}; CNHOR_{d} -CH(NHCH₂CH₂CH₂OH)-, -CH(NHPh)- and -CH(CH₃)- and in particular a radical -CO-, -CH(OH)- or -CH(NH₂)- and more particularly a radical -CH(OH)-,
- R' denotes:
   * a hydrogen atom;
   a saturated linear C1-C18 alkyl radical,
   * an unsaturated linear C2-C18 alkyl radical,
   * a saturated or unsaturated branched C3-C18 alkyl radical,
   * a saturated or unsaturated C5 or C6 cyclic radical,
   * a linear or branched, saturated or unsaturated C2-C18, or saturated or unsaturated C5
   or C6 cyclic acyl radical.

**Rₐ** denotes:
- a hydrogen atom
- a linear or branched C1-C4 alkyl radical, or a linear or branched unsaturated C3-C4 hydrocarbon-based radical
- a linear or branched C2-C18 acyl or linear or branched C2-C18 alkenylcarbonyl radical

**R_{b}** denotes:
- a hydrogen atom
- a linear C2-C18 or branched C3-C18 alkyl radical, or a linear or branched unsaturated C3-C4 hydrocarbon-based radical or a radical -CH(Z₁)-CO₂Z₂ in which
   Z₁ denotes a hydrogen atom or a linear or branched C1-C6, or saturated or unsaturated cyclic C3-C6 alkyl radical, the said radical being optionally substituted with at least one group chosen from =NH, -NH₂, -N(T)₂, =O, -OH, -OT, -SH, -ST, -CO₂T, phenyl, phenyl substituted with -OH or -OT, 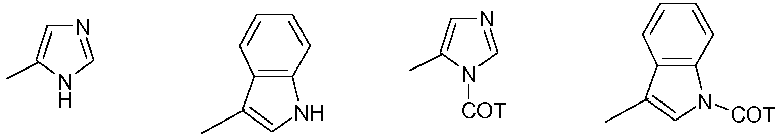 and/or interrupted with a group -NH-, -N-(COT)- or -S- with T denoting a linear or branched C1-C6 or cyclic C3-C6 alkyl radical.
   and Z₂ denotes a hydrogen atom or a linear or branched C1-C6 alkyl radical

**R_{c}** denotes:
- a hydrogen atom
- a linear C1-C4 or branched C3-C4 alkyl radical, or a linear or branched unsaturated C3-C4 hydrocarbon-based radical, the said radical being optionally substituted with a phenyl group.

**Rd** denotes:
- a hydrogen atom
- a linear C1-C18 or branched C3-C18 alkyl radical, or a linear or branched unsaturated C3-C18 hydrocarbon-based radical, the said radical being optionally substituted with a phenyl group,
the bond S-CH₂-X represents a bond of C-anomeric nature, which may be α or β,
and also the cosmetically acceptable salts thereof, solvates thereof such as hydrates, and optical and geometrical isomers thereof.

The C-glycosides of formula (I) that are useful for performing the invention are in particular those for which R denotes a saturated linear C₁-C₆, in particular C₁-C₄ and preferentially C₁-C₂ alkyl radical and more preferentially a methyl radical.
Among the alkyl groups that are suitable for use in the invention, mention may be made especially of methyl, ethyl, isopropyl, n-propyl, n-butyl, t-butyl, isobutyl, sec-butyl, pentyl, n-hexyl, cyclopropyl, cyclopentyl and cyclohexyl groups.

According to one embodiment of the invention, a C-glycoside compound may be used corresponding to formula (I) for which S may represent a monosaccharide or a polysaccharide comprising up to 6 sugar units, in pyranose and/or furanose form and of L and/or D series, the said mono- or polysaccharide containing at least one mandatorily free hydroxyl function and/or optionally one or more mandatorily protected amine functions, X and R otherwise conserving all the definitions given previously.

Advantageously, a monosaccharide of the invention may be chosen from D-glucose, D-galactose, D-mannose, D-xylose, D-lyxose, L-fucose,
L-arabinose, L-rhamnose, D-glucuronic acid, D-galacturonic acid,
D-iduronic acid, N-acetyl-D-glucosamine and N-acetyl-D-galactosamine and advantageously denotes D-glucose, D-xylose, N-acetyl-D-glucosamine or L-fucose, and in particular D-xylose.

More particularly, a polysaccharide of the invention containing up to 6 sugar units may be chosen from D-maltose, D-lactose, D-cellobiose,
D-maltotriose, a disaccharide combining a uronic acid chosen from D-iduronic acid and D-glucuronic acid with a hexosamine chosen from D-galactosamine, D-glucosamine, N-acetyl-D-galactosamine and N-acetyl-D-glucosamine, an oligosaccharide containing at least one xylose which may be advantageously chosen from xylobiose, methyl-p-xylobioside, xylotriose, xylotetraose, xylopentaose and xylohexaose and especially xylobiose, which is composed of two xylose molecules linked via a 1-4 bond.

More particularly, S may represent a monosaccharide chosen from D-glucose, D-xylose, L-fucose, D-galactose and D-maltose, and especially D-xylose.

The acceptable salts of the compounds described in the present invention include conventional non-toxic salts of the said compounds, such as those formed from organic or mineral acids. By way of example, mention may be made of the salts of mineral acids, such as sulfuric acid, hydrochloric acid, hydrobromic acid, hydriodic acid, phosphoric acid or boric acid. Mention may also be made of the salts of organic acids, which may comprise one or more carboxylic, sulfonic or phosphonic acid groups. They may be linear, branched or cyclic aliphatic acids, or alternatively aromatic acids. These acids may also include one or more heteroatoms chosen from O and N, for example in the form of hydroxyl groups. Mention may be made especially of propionic acid, acetic acid, terephthalic acid, citric acid and tartaric acid.

When the compound of formula (I) comprises an acidic group, neutralization of the acid group(s) may be performed with a mineral base, such as LiOH, NaOH, KOH, Ca(OH)₂, NH₄OH, Mg(OH)₂ or Zn(OH)₂; or with an organic base such as a primary, secondary or tertiary alkylamine, for example triethylamine or butylamine. This primary, secondary or tertiary alkylamine may comprise one or more nitrogen and/or oxygen atoms and may thus comprise, for example, one or more alcohol functions; mention may be made especially of 2-amino-2-methylpropanol, triethanolamine, 2-dimethylaminopropanol and 2-amino-2-(hydroxymethyl)-1,3-propanediol. Mention may also be made of lysine or 3-(dimethylamino)propylamine.

The solvates that are acceptable for the compounds described in the present invention comprise conventional solvates such as those formed during the final step of preparation of the said compounds due to the presence of solvents. Mention may be made, by way of example, of the solvates due to the presence of water or of linear or branched alcohols, such as ethanol or isopropanol.

According to a first embodiment, preferentially, use is made of a C-glycoside derivative of formula (I) in which:
- R denotes an unsubstituted linear C₁-C₄ alkyl radical, especially C₁-C₂, in particular methyl;
- S represents a monosaccharide as described previously, chosen in particular from D-glucose, D-xylose, N-acetyl-D-glucosamine and
   L-fucose, and in particular D-xylose;
- X represents a group chosen from -CO-, -CH(OH)- and -CH(NH₂)- and preferentially a group -CH(OH)-.

As non-limiting illustrations of the C-glycoside compounds that are more particularly suitable for use in the invention, mention may be made especially of the following compounds:
- C-β-D-xylopyranoside-n-propane-2-one,
- C-α-D-xylopyranoside-n-propan-2-one,
- C-β-D-xylopyranoside-2-hydroxypropane,
- C-α-D-xylopyranoside-2-hydroxypropane,
- 1-(C-β-D-fucopyranoside)propan-2-one,
- 1-(C-α-D-fucopyranoside)propan-2-one,
- 1-(C-β-L-fucopyranoside)propan-2-one,
- 1-(C-α-L-fucopyranoside)propan-2-one,
- 1-(C-β-D-fucopyranoside)-2-hydroxypropane,
- 1-(C-α-D-fucopyranoside)-2-hydroxypropane,
- 1-(C-β-L-fucopyranoside)-2-hydroxypropane,
- 1-(C-α-L-fucopyranoside)-2-hydroxypropane,
- 1-(C-β-D-glucopyranosyl)-2-hydroxypropane,
- 1-(C-α-D-glucopyranosyl)-2-hydroxypropane,
- 1-(C-β-D-galactopyranosyl)-2-hydroxypropane,
- 1-(C-α-D-galactopyranosyl)-2-hydroxypropane,
- 1-(C-β-D-fucofuranosyl)propan-2-one,
- 1-(C-α-D-fucofuranosyl)propan-2-one,
- 1-(C-β-L-fucofuranosyl)propan-2-one,
- 1-(C-α-L-fucofuranosyl)propan-2-one,
- C-β-D-maltopyranoside-n-propane-2-one,
- C-α-D-maltopyranoside-n-propan-2-one,
- C-β-D-maltopyranoside-2-hydroxypropane,
- C-α-D-maltopyranoside-2-hydroxypropane, isomers thereof and mixtures thereof.

According to one embodiment, C-β-D-xylopyranoside-2-hydroxypropane or C-α-D-xylopyranoside-2-hydroxypropane, and preferably C-β-D-xylopyranoside-2-hydroxypropane, may be advantageously used for the preparation of a composition according to the invention.

According to a particular embodiment, the C-glycoside compound may be C-β-D-xylopyranoside-2-hydroxypropane (or hydroxypropyltetrahydropyrantriol) in the form of a solution containing 30% by weight of active material in a water/propylene glycol mixture (60%/40% by weight).

A C-glycoside derivative that is suitable for use in the invention may especially be obtained via the synthetic method described in document WO 02/051 828.

According to a second embodiment, the C-glycosides are preferably chosen from the C-glycosides of general formula (I) in which S is xylose and R represents a saturated or unsaturated C2 alkyl radical substituted with a radical Y which denotes a phenyl radical or a heterocycle, optionally substituted with 1 to 5 groups (ORₐ) and represented by formula (II) below: 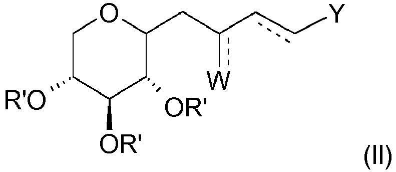 in which:
- the compounds of formula (I) are xylose derivatives
- Y denotes a phenyl radical or a heterocycle, optionally substituted with 1 to 5 groups (ORₐ)
- W = -OR'; (=O); NR_{b}R_{c}; NHOR_{d}
- R' denotes:
   * a hydrogen atom;
   * a saturated linear C1-C18 alkyl radical,
      * an unsaturated linear C2-C18 alkyl radical,
   * a saturated or unsaturated branched C3-C18 alkyl radical,
   * a saturated or unsaturated C5 or C6 cyclic radical,
   * a linear or branched, saturated or unsaturated C2-C18, or saturated or unsaturated C5 or C6 cyclic acyl radical.

**Rₐ** denotes:
- a hydrogen atom
- a linear or branched C1-C4 alkyl radical, or a linear or branched unsaturated C3-C4 hydrocarbon-based radical
- a linear or branched C2-C18 acyl or linear or branched C2-C18 alkenylcarbonyl radical

When Y denotes a phenyl radical or a heterocycle substituted with 2 to 5 groups (ORₐ), two adjacent groups ORₐ may together form a divalent radical -O-CH₂-O
with the proviso that when W = OH, the compound does not comprise an ethylenic double bond alpha to the carbon bearing this OH.

**R_{b}** denotes:
- a hydrogen atom
- a linear C2-C18 or branched C3-C18 alkyl radical, or a linear or branched unsaturated C3-C4 hydrocarbon-based radical or a radical -CH(Z₁)-CO₂Z₂ in which Z₁ denotes a hydrogen atom or a linear or branched C1-C6, or saturated or unsaturated C3-C6 cyclic alkyl radical, the said radical being optionally substituted with at least one group chosen from =NH, -NH₂, -N(T)₂, =O, -OH, -OT, -SH, -ST, -CO₂T, phenyl, phenyl substituted with -OH or -OT, 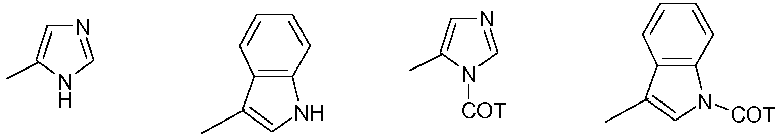
and/or interrupted with a group -NH-, -N-(COT)- or -S- with T denoting a linear or branched C1-C6 or cyclic C3-C6 alkyl radical.
and Z₂ denotes a hydrogen atom or a linear or branched C1-C6 alkyl radical

**R_{c}** denotes:
- a hydrogen atom
- a linear C1-C4 or branched C3-C4 alkyl radical, or a linear or branched unsaturated C3-C4 hydrocarbon-based radical, the said radical being optionally substituted with a phenyl group.

**Rd** denotes:
- a hydrogen atom
- a linear C1-C18 or branched C3-C18 alkyl radical, or a linear or branched unsaturated C3-C18 hydrocarbon-based radical, the said radical being optionally substituted with a phenyl group,
and also the cosmetically acceptable salts thereof, solvates thereof such as hydrates, and stereoisomers thereof.

For the purposes of the present invention, the term "heterocycle" denotes a saturated or unsaturated 5- to 10-membered cyclic hydrocarbon-based radical, including an aromatic radical, comprising at least one heteroatom chosen from O, S and N. Preferably, heterocycle denotes a pyridine, pyrimidine or indole radical and more preferentially pyridine or indole.

The preferred compounds of formula (II) are those for which:
- the compounds of formula (II) are xylose derivatives
- Y denotes a phenyl radical or a heterocycle, optionally substituted with 1 to 5 groups (ORₐ)
- W = -OR'; (=O); NR_{b}R_{c}; NHOR_{d}
- R' denotes:
   * a hydrogen atom,
   * a linear or branched, saturated or unsaturated C1-C12, or saturated cyclic C5 or C6 alkyl radical,
   * a linear or branched, saturated or unsaturated C1-C6, or saturated cyclic C5 or C6 acyl radical,

**Rₐ** denotes:
- a hydrogen atom
- a linear or branched C1-C4 alkyl radical,
- a linear or branched C1-C6 acyl radical,

When Y denotes a phenyl radical or a heterocycle substituted with 2 to 5 groups (ORₐ), two adjacent groups ORₐ may together form a divalent radical -O-CH₂-O
with the proviso that when W = OH, the compound does not comprise an ethylenic double bond alpha to the carbon bearing this OH.

**R_{b}** denotes:
- a hydrogen atom
- a linear C2-C12 or branched C3-C12 alkyl radical, or a radical -CH(Z₁)-CO₂Z₂ in which
   Z₁ denotes a hydrogen atom or a linear or branched C1-C6, or saturated or unsaturated cyclic C3-C6 alkyl radical, the said radical being optionally substituted with at least one group chosen from =NH, -NH₂, -N(T)₂, =O, -OH, -OT, -SH, -ST, -CO₂T, phenyl, phenyl substituted with -OH or -OT, 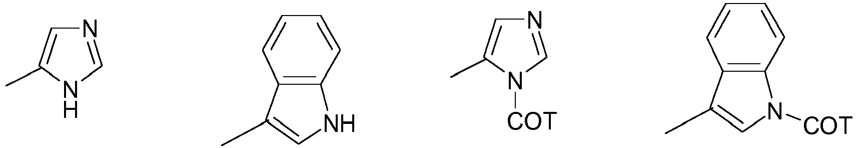
and/or interrupted with a group -NH-, -N-(COT)- or -S- with T denoting a linear C1-C6 or cyclic C5-C6 alkyl radical,
and Z₂ denotes a hydrogen atom or a linear C1-C6 alkyl radical

**R_{c}** denotes:
- a hydrogen atom
- a linear C1-C4 or branched C3-C4 alkyl radical, or a linear or branched unsaturated C3-C4 hydrocarbon-based radical, the said radical being optionally substituted with a phenyl group.

**Rd** denotes:
- a hydrogen atom
- a linear C1-C12 or branched C3-C12 alkyl radical, the said radical being optionally substituted with a phenyl group,
and also the cosmetically acceptable salts thereof, solvates thereof such as hydrates, and stereoisomers thereof.

For the purposes of the present invention, the term "heterocycle" denotes a saturated or unsaturated 5- to 10-membered cyclic hydrocarbon-based radical, including an aromatic radical, comprising at least one heteroatom chosen from O, S and N. Preferably, heterocycle denotes a pyridine, pyrimidine or indole radical and more preferentially pyridine or indole.

The compounds of formula (II) that are particularly preferred are those for which:
- the compounds of formula (II) are xylose derivatives
- Y denotes a phenyl radical or a heterocycle, optionally substituted with 1 to 3 groups (ORₐ)
- W = -OR'; (=O); NR_{b}R_{c}; NHOR_{d}
- R' denotes:
   * a hydrogen atom,
   * a linear or branched, saturated or unsaturated C1-C4 alkyl radical,
   * a linear or branched C1-C6 acyl radical.

**Rₐ** denotes:
- a hydrogen atom
- a linear or branched C1-C4 alkyl radical,
- a linear or branched C1-C6 acyl radical,
when Y denotes a phenyl radical or a heterocycle substituted with 2 or 3 groups (ORₐ), two adjacent groups ORₐ may together form a divalent radical -O-CH₂-O
with the proviso that when W = OH, the compound does not comprise an ethylenic double bond alpha to the carbon bearing this OH.

**R_{b}** denotes:
- a hydrogen atom
- a linear C2-C8 or branched C3-C8 alkyl radical, or a radical -CH(Z₁)-CO₂Z₂ in which Z₁ denotes a hydrogen atom or a linear or branched C1-C6, or saturated or unsaturated cyclic C3-C6 alkyl radical, the said radical being optionally substituted with at least one group chosen from =NH, -NH₂, -N(T)₂, =O, -OH, -OT, -SH, -ST, -CO₂T, phenyl, phenyl substituted with -OH or -OT, 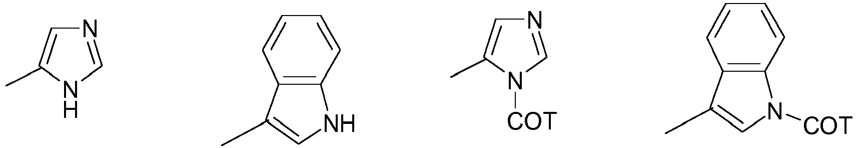
and/or interrupted with a group -NH-, -N-(COT)- or -S- with T denoting a linear C1-C6 or cyclic C5-C6 alkyl radical,
and Z₂ denotes a hydrogen atom or a linear C1-C6 alkyl radical

**R_{c}** denotes:
- a hydrogen atom
- a linear C1-C4 or branched C3-C4 alkyl radical, optionally substituted with a phenyl group.

**Rd** denotes:
- a hydrogen atom
- a linear C1-C4 or branched C3-C4 alkyl radical, the said radical being optionally substituted with a phenyl group,
and also the cosmetically acceptable salts thereof, solvates thereof such as hydrates, and stereoisomers thereof.

For the purposes of the present invention, the term "heterocycle" denotes a saturated or unsaturated 5- to 10-membered cyclic hydrocarbon-based radical, including an aromatic radical, comprising at least one heteroatom chosen from O, S and N. Preferably, heterocycle denotes a pyridine, pyrimidine or indole radical and more preferentially pyridine or indole.

The compounds of formula (II) that are more particularly preferred are the following:
**Compound 1.** : (3R,4S,5R)-2-[2-hydroxy-4-(4-hydroxy-3-methoxyphenyl)butyl]tetrahydro-2H-pyran-3,4, 5-triol 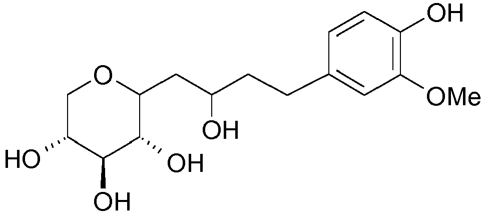
**Compound 2.** 4-(4-hydroxy-3-methoxyphenyl)-1-[(3R,4S,5R)-3,4,5-trihydroxytetrahydro-2H-pyran-2-yl butan-2-one 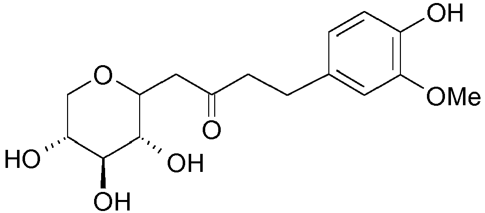
**Compound 3.** (3R,4S,5R)-2-[2-hydroxy-4-(4-hydroxyphenyl)butyl]tetrahydro-2H-pyran-3,4,5-triol 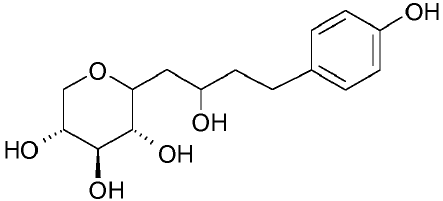
**Compound 4.** 4-(4-hydroxyphenyl)-1-[(3R,4S,5R)-3,4,5-trihydroxytetrahydro-2H-pyran-2-yl]butan-2-on e 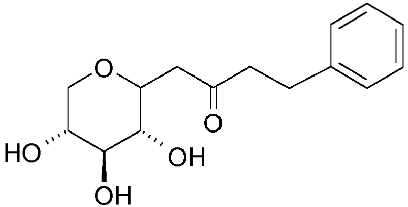
**Compound 5.** (3R,4S,5R)-2-[2-(benzylamino)-4-(4-hydroxy-3-methoxyphenyl)butyl]tetrahydro-2H-pyr an-3,4,5-triol 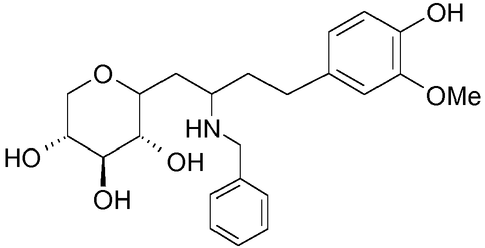
**Compound 6.**
   ethyl {[3-(4-hydroxy-3-methoxyphenyl)-1-{[(3R,4S,5R)-3,4,5-trihydroxytetrahydro-2H-pyran-2 -yl]methyl}propyl]amino}(phenyl)acetate 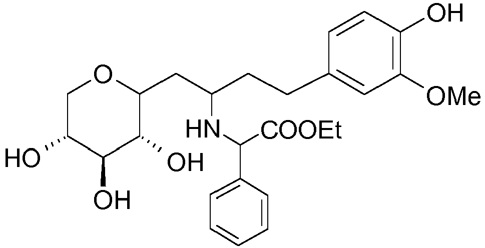
**Compound** 7. (3E)-4-phenyl-1-[(3R,4S,5R)-3,4,5-trihydroxytetrahydro-2H-pyran-2-yl]but-3-en-2-one 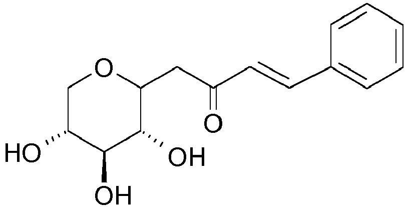
**Compound 8.** (3E)-4-(4-hydroxy-3,5-dimethoxyphenyl)-1-[(3R,4S,5R)-3,4,5-trihydroxytetrahydro-2H-p yran-2-yl]but-3-en-2-one 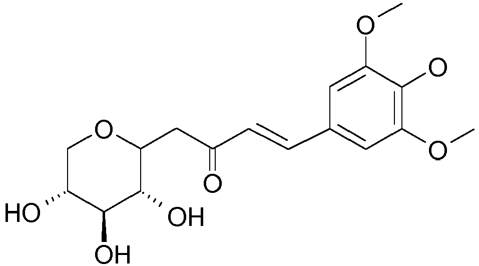
< **Compound 9.** (3R,4S,5R)-2-[2-hydroxy-4-(2-hydroxyphenyl)butyl]tetrahydro-2H-pyran-3,4,5-triol 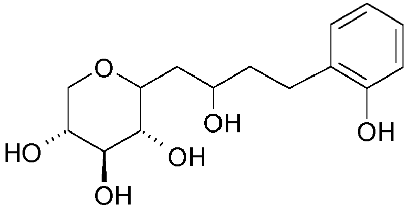
**Compound 10:** (3R,4S,5R)-2-[2-hydroxy-4-(3-hydroxy-4-methoxyphenyl)butyl]tetrahydro-2H-pyran-3,4, 5-triol 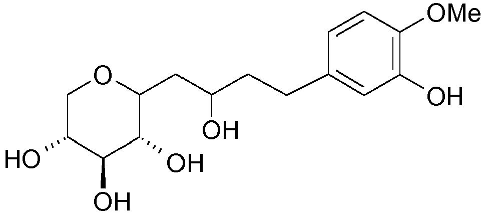
**Compound 11.** (3R,4S,5R)-2-[2-hydroxy-4-(2,4-di-hydroxyphenyl)butyl]tetrahydro-2H-pyran-3,4,5-triol 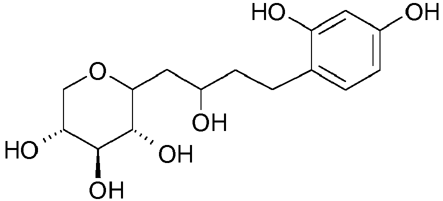
**Compound 12.** (3R,4S,5R)-2-[2-hydroxy-4-(3-ethoxy-4-hydroxyphenyl)butyl]tetrahydro-2H-pyran-3,4,5-triol 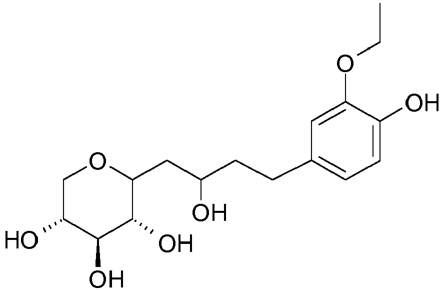
**Compound 13.** 5,9-anhydro-1,2,4-trideoxy-1-pyridin-3-yl-D-xylononitol 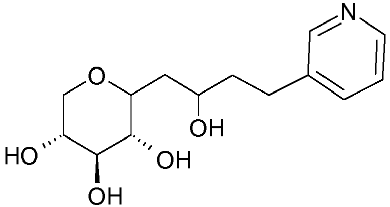
**Compound 14.** (3R,4S,5R)-2-[(2E)-2-(methoxyimino)-4-phenylbutyl]tetrahydro-2H-pyran-3,4,5-triol 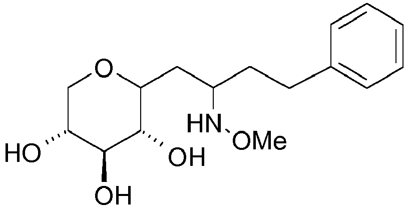
**Compound 15.** 5,9-anhydro-1,2,4-trideoxy-7-O-pentanoyl-1-phenyl -D-xylonon-3-ulose 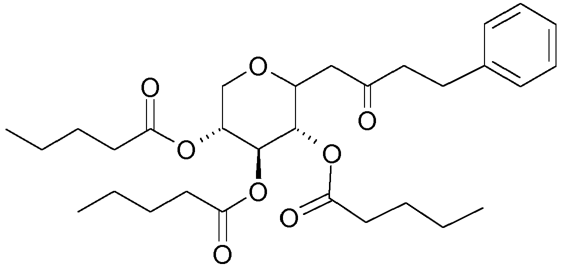
**Compound 16.** 5,9-anhydro-1,2,4-trideoxy-1-(3,4,5-trimethoxyphenyl)-D-xylononitol

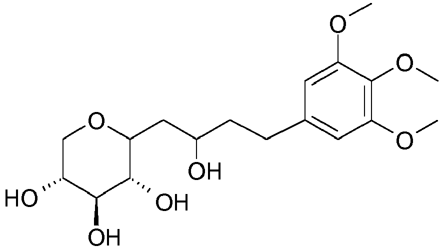

The C-glycoside compound (c) of formula (II) is preferably chosen from compounds (1) and (11).

According to the invention, the C-glycoside compound (c) is preferably chosen from C-β-D-xylopyranoside-2-hydroxypropane, -α-D-xylopyranoside-2-hydroxypropane, compounds (1) and (11) and more particularly from C-β-D-xylopyranoside-2-hydroxypropane and compound 1.

The compounds of formula (I) and/or (II) may be synthesized according to the general procedure given in patent application EP 2 376 510 or according to patent application FR 1 262 731.

Needless to say, according to the invention, a C-glycoside derivative corresponding to formula (I) and/or (II) may be used alone or as a mixture with other C-glycoside derivatives and in any proportion.

The amount of the C-glycoside compound (c) in the cosmetic composition according to the present invention may range from 0.01% to 20% by weight, preferably from 0.1% to 15% by weight and more preferably from 1% to 10% by weight relative to the total weight of the composition.

### [Water]

The cosmetic composition according to the present invention comprises water.

The amount of water is not limited, and may range from 50% to 99% by weight, preferably from 55% to 95% by weight and more preferably from 60% to 90% by weight relative to the total weight of the composition.

### [Additional surfactant]

The cosmetic composition according to the present invention may also comprise at least one nonionic surfactant other than (b) above and/or at least one additional ionic surfactant.

As additional surfactant, use may be made of at least one nonionic surfactant other than b), in particular with an HLB value of less than 8.0 or more than 14.0.

As additional nonionic surfactant, mention may be made of compounds of the type listed in (b) above except that the additional nonionic surfactant has an HLB value of less than 8.0, preferably less than 9.0 and more preferably less than 10.0, and greater than 14.0, preferably greater than 13.5 and more preferably greater than 13.0.

As additional surfactant, use may be made of at least one ionic surfactant. The ionic surfactant may be chosen from cationic surfactants, anionic surfactants and amphoteric surfactants, and is more particularly chosen from anionic surfactants.

According to one embodiment, the cationic surfactant that may be used in the compositions of the invention is chosen from quaternary ammonium salts, for example behenyltrimethylammonium chloride, cetyltrimethylammonium chloride, Quaternium-83, Quaternium-87, Quaternium-22, behenylamidopropyl-2,3-dihydroxypropyldimethylammonium chloride, palmitylamidopropyltrimethylammonium chloride and stearamidopropyldimethylamine.

### Anionic surfactant

The anionic surfactants may comprise at least one group chosen from carboxylic, sulfate, sulfonate and phosphate groups. Use may be made of alkyl phosphates, alkyl sulfosuccinates, amino acid derivatives, alkyl sulfates, alkyl ether sulfates, alkylsulfonates, alkyl isethionates, alkyl taurates, alkyl sulfoacetates, alkylpolypeptides and anionic alkylpolyglucoside derivatives, and mixtures thereof, the alkyl groups containing from 8 to 22 carbon atoms.
1) As alkyl phosphates, examples that may be mentioned include monoalkyl phosphates and dialkyl phosphates, such as the lauryl monophosphate sold under the name MAP 20® by Kao Chemicals, the potassium salt of dodecyl phosphate, the mixture of monoesters and diesters (mainly a diester) sold under the name Crafol AP-31® by Cognis, the mixture of octyl phosphate monoester and diester, sold under the name Crafol AP-20® by Cognis, the mixture of ethoxylated (7 mol of EO) 2-butyloctyl phosphate monoester and diester, sold under the name Isofol 12 7 EO-Phosphate Ester® by Condea, the potassium or triethanolamine salt of mono(C₁₂-C₁₃ alkyl) phosphate, sold under the references Arlatone MAP 230K-40® and Arlaton MAP 230T-60® by Uniqema, the potassium lauryl phosphate sold under the name Dermalcare MAP XC-99/09® by Rhodia Chimie, and the potassium cetyl phosphate sold under the name Arlatone MAP 160K by Uniqema.
2) As surfactant comprising at least one carboxylic group, mention may be made of:
   - alkylamido ether carboxylates (AEC), such as sodium laurylamido ether carboxylate (3 EO), sold under the name Akypo Foam 30® by Kao Chemicals;
   - alkyl ether carboxylic acid salts, such as the oxyethylenated sodium lauryl ether carboxylate sold under the name Akypo Soft 45 NV® by Kao Chemicals, oxyethylene-oxyethylene-polyoxyethylene and carboxymethyl fatty acids originating from olive oil, sold under the name Olivem 400® by Biologia E Tecnologia, or the oxyethylenated sodium tridecyl ether carboxylate, sold under the name Nikkol ECTD-6NEX® by Nikkol; and
   - fatty acid salts (soaps) bearing a C₆ to C₂₂ alkyl chain, which are neutralized with an organic or mineral base such as potassium hydroxide, sodium hydroxide, triethanolamine, N-methylglucamine, lysine or arginine.
3) Mention may be made in particular of amino acid derivatives such as:
   - (C6-C18)alkyl sarcosinates, such as sodium lauroyl sarcosinate, sold under the name Sarkosyl NL 97® by Ciba or sold under the name Oramix L 30® by SEPPIC, sodium myristoyl sarcosinate, sold under the name Nikkol Sarcosinate MN® by Nikkol, or sodium palmitoyl sarcosinate, sold under the name Nikkol Sarcosinate PN® by Nikkol;
   - (C6-C18)alkyl alaninates, such as sodium N-lauroyl-N-methylamidopropionate, sold under the name Sodium Nikkol Alaninate LN 30® by Nikkol or sold under the name Alanone ALE® by Kawaken, or triethanolamine-N-lauroyl-N-methylalanine, sold under the name Alanone ALTA® by Kawaken;
   - (C6-C18)alkyl glutamates, such as triethanolamine monococoyl glutamate, sold under the name Acylglutamate CT-12® by Ajinomoto, triethanolamine lauroyl glutamate, sold under the name Acylglutamate LT-12® by Ajinomoto;
   - (C6-C18)alkyl aspartates, such as the mixture of triethanolamine-N-lauroyl aspartate and triethanolamine-N-myristoyl aspartate, sold under the name Asparack® by Mitsubishi;
   - (C6-C18) alkyl glycinates, such as sodium N-cocoyl glycinate, sold under the names Amilite GCS-12® and Amilite GCK 12 by Ajinomoto;
   - (C6-C18)alkyl citrates, such as the citric monoester of oxyethylene-oxyethylene-oxyethylene (9 mol) coconut alcohols, sold under the name Witconol EC 1129 by Goldschmidt; and
   - (C6-C18)alkyl galacturonates, such as sodium dodecyl-D-galactoside-uronate, sold by Soliance.
4) As (C6-C18) alkyl methyltaurates, mention may be made of the sodium salt of palm oil methyltaurate, sold under the name Hostapon CT Paté® by Clariant;
   N-acyl-N-methyltaurates such as sodium N-cocoyl-N-methyltaurate, sold under the name Hostapon LT-SF® by Clariant or sold under the name Nikkol CMT-30-T® by Nikkol, or sodium palmitoyl methyltaurate, sold under the name Nikkol PMT® by Nikkol.
5) The anionic derivatives of alkyl polyglucosides may in particular be citrates, tartrates, sulfosuccinates, carbonates and glycerol ethers obtained from alkyl polyglucosides. Mention may be made, for example, of the sodium salt of cocoylpolyglucoside (1,4) tartaric ester, sold under the name Eucarol AGE-ET® by Cesalpinia, the disodium salt of cocoylpolyglucoside (1,4) sulfosuccinic ester, sold under the name Essai 512 MP® by SEPPIC, or the sodium salt of cocoylpolyglucoside (1,4) citric ester, sold under the name Eucarol AGE-EC® by Cesalpinia.

It is preferable for the amino acid derivatives to be acylglycine derivatives, in particular an acylglycine salt.

The acylglycine derivatives may be chosen from acylglycine salts (or acylglycinates) or glycine salts (or glycinates), and in particular the following:
i) Acylglycinates of formula (I):

   R-HNCH₂COOX (I)

   in which:
   - R represents an acyl group R'C=O, with R' representing a linear or branched, saturated or unsaturated hydrocarbon-based chain, preferably comprising from 10 to 30 carbon atoms, preferably from 12 to 22 carbon atoms, preferably from 14 to 22 carbon atoms and more preferably from 16 to 20 carbon atoms, and
   - X represents a cation chosen, for example, from ions of alkali metals, such as Na, Li or K, preferably Na or K, ions of alkaline-earth metals such as Mg, and ammonium groups, and mixtures thereof.

The acyl group may be chosen in particular from lauroyl, myristoyl, behenoyl, palmitoyl, stearoyl, isostearoyl, olivoyl, cocoyl and oleoyl groups, and mixtures thereof.

Preferably, R is a cocoyl group.

### ii) Glycinates of formula (II) below:

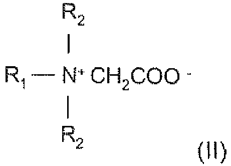

in which:
- R₁ represents a linear or branched, saturated or unsaturated hydrocarbon-based chain, comprising from 10 to 30 carbon atoms, preferably from 12 to 22 carbon atoms and more preferably from 16 to 20 carbon atoms; R₁ is advantageously chosen from lauryl, myristyl, palmityl, stearyl, cetyl, cetearyl and oleyl groups, and mixtures thereof, and preferably stearyl and oleyl groups,
- the groups R₂, which are identical or different, represent a group R"OH, R" being an alkyl group comprising from 2 to 10 carbon atoms and preferably from 2 to 5 carbon atoms.

As compound of formula (I), mention may be made, for example, of the compounds having the INCI name sodium cocoyl glycinate, for instance Amilite GCS-12, sold by Ajinomoto, or potassium cocoyl glycinate, for instance Amilite GCK-12 from Ajinomoto.

As compounds of formula (II), use may be made of dihydroxyethyloleyl glycinate or dihydroxyethylstearyl glycinate.

The amount of the additional surfactant(s) may be from 0.01% by weight to 20% by weight, preferably from 0.10% by weight to 10% by weight and more preferably from 1% by weight to 5% by weight, relative to the total weight of the composition.

### [Polyol]

The composition according to the present invention may also comprise at least one polyol.

According to the present invention, the term "polyol" denotes a compound bearing two or more than two hydroxyl groups.

The polyol may be a C₂-C₁₂ polyol, preferably a C₂₋₉ polyol, comprising at least 2 hydroxyl groups and preferably 2 to 5 hydroxyl groups.

The polyol may be a natural or synthetic polyol. The polyol may have a linear, branched or cyclic molecular structure.

The polyol may be chosen from glycerol and derivatives thereof, and glycols and derivatives thereof. The polyol may be chosen from the group consisting of glycerol, diglycerol, polyglycerol, diethylene glycol, propylene glycol, dipropylene glycol, butylene glycol, pentylene glycol, hexylene glycol, 1,3-propanediol, 1,5-pentanediol, polyethylene glycols, especially containing from 5 to 50 ethylene oxide groups, and sugars such as sorbitol.

The polyol may be present in an amount in the range from 0.01% to 30% by weight and preferably from 0.1% to 20% by weight, such as from 1% to 10% by weight, relative to the total weight of the composition.

### [Thickener]

The cosmetic composition according to the present invention may also comprise at least one thickener.

The thickener may be chosen from organic and mineral thickeners.

The thickener is preferably chosen from associative thickeners and polysaccharides such as starch and xanthan gum.

In the present context, the term "associative thickener" denotes an amphiphilic thickener comprising both hydrophilic and hydrophobic units, for example comprising at least one C₈-C₃₀ fatty chain and at least one hydrophilic unit.

The viscosity of the cosmetic composition according to the present invention is not particularly limited. The viscosity may be measured at 25°C with viscometers or rheometers, preferably having cone-plate geometry. Preferably, the viscosity of the cosmetic composition according to the present invention may be, for example, from 1 to 2000 Pa.s and preferably from 1 to 1000 Pa.s at 25°C and 1 s⁻¹.

The thickener may be present in an amount in the range from 0.001% to 10% by weight and preferably from 0.01 % to 10% by weight, for example from 0.1 % to 5% by weight, relative to the total weight of the composition.

### [Other components]

The cosmetic composition according to the present invention may also comprise an efficient amount of other components, previously known elsewhere in compositions, especially cosmetic compositions, such as various adjuvants, anti-ageing agents, bleaching agents, anti-greasy skin agents, sequestrants such as EDTA and etidronic acid, UV stabilizers, preserving agents, vitamins or provitamins, for example panthenol, opacifiers, fragrances, plant extracts, cationic polymers, etc.

The cosmetic composition according to the present invention may also comprise at least one organic solvent. Consequently, the organic solvent is preferably water-miscible. Examples of organic solvents that may be mentioned include C₂-C₄ alkanols, such as ethanol and isopropanol; aromatic alcohols such as benzyl alcohol and phenoxyethanol; similar products; and mixtures thereof.

The water-soluble organic solvents may be present in a content ranging from 0 to 20% by weight, preferably from 0.1% to 10% by weight and more preferably from 0.5% to 5% by weight relative to the total weight of the composition.

### [Preparation and properties]

The cosmetic composition according to the present invention may be prepared by mixing the essential and optional components above according to a conventional process. The conventional process comprises mixing with a high-pressure homogenizer (a high-energy process). As a variant, the cosmetic composition may be prepared via a low-energy process such as a phase inversion temperature (PIT) process, a phase inversion concentration (PIC), self-emulsification, and the like. Preferably, the cosmetic composition is prepared via a low-energy process.

The ratio of the nonionic surfactant (b) to the oil (a) may be from 0.25 to 6, preferably from 0.3 to 3 and more preferably from 0.4 to 1.5. In particular, the ratio of the nonionic surfactant (b) to the oil (a) is preferably 1.5 or less, for example from 0.25 to 1.5, preferably from 0.3 to 1.5 and more preferably from 0 to 1.5.

The cosmetic composition according to the present invention is in the form of a nanoemulsion or microemulsion.

The term "microemulsion" may be defined in two ways, i.e. in a broad sense and in a narrower sense. Namely, in one case ("microemulsion in the narrow sense"), the term microemulsion denotes a thermodynamically stable isotropic single liquid phase containing a ternary system having three components comprising an oily component, an aqueous component and a surfactant, and, in the other case ("microemulsion in the broad sense"), among the thermodynamically unstable typical emulsion systems, the term microemulsion also comprises emulsions that have transparent or translucent appearances on account of their smaller particle sizes (Satoshi Tomomasa, et al., Oil Chemistry, vol. 37, No. 11 (1988), pp. 48-53). In the present context, the term "microemulsion" denotes a "microemulsion in the narrow sense", i.e. a thermodynamically stable isotropic single liquid phase.

The microemulsion denotes a state of a microemulsion of O/W (oil-in-water) type in which the oil is dissolved by micelles, a microemulsion of W/O (water-in-oil) type in which the water is dissolved by inverse micelles, or a bicontinuous microemulsion in which the number of associations of surfactant molecules tends to infinity such that the aqueous phase and the oily phase both have a continuous structure.

The microemulsion may have a dispersed phase with a numerical mean diameter of 100 nm or less, preferably 50 nm or less and more preferably 20 nm or less, measured by laser particle size analysis.

The term "nanoemulsion" presently denotes an emulsion characterized by a dispersed phase with a size of less than 350 nm, the dispersed phase being stabilized by a crown of the nonionic surfactant (b) which may optionally form a liquid crystal phase of lamellar type, at the dispersed phase/continuous phase interface. In the absence of specific opacifiers, the transparency of nanoemulsions is due to the small size of the dispersed phase, this small size being able to be obtained by means of using mechanical energy and in particular a high-pressure homogenizer.

Nanoemulsions may be distinguished from microemulsions by their structure. Specifically, microemulsions are thermodynamically stable dispersions formed, for example, from swollen micelles of nonionic surfactant (b) with the oil (a). Furthermore, microemulsions do not require substantial mechanical energy to be prepared.

The microemulsion may have a dispersed phase with a numerical mean diameter of 300 nm or less, preferably 200 nm or less and more preferably 100 nm or less, measured by laser particle size analysis.

The cosmetic composition according to the present invention may be in the form of an O/W nanoemulsion or microemulsion, a W/O nanoemulsion or microemulsion, or a bicontinuous emulsion. It is preferable for the cosmetic composition according to the present invention to be in the form of an O/W nanoemulsion or microemulsion.

It is preferable for the cosmetic composition according to the present invention to be in the form of an O/W emulsion.

The mean size of the droplets of the oily phase is measured by dynamic light scattering (DLS) with a Vasco particle size analyser.
These measurements are taken on the undiluted emulsion.
The numerical mean size (µm) of the droplets of oily phase of the composition of the invention is less than 300 nm, preferably from 10 nm to 150 nm and more preferably from 20 nm to 100 nm.

The cosmetic composition according to the present invention may have a transparent or slightly translucent appearance, preferably a transparent appearance.

The transparency may be measured by measuring the transmission factor with an absorption spectrometer in the visible region (for example, the transparency is measured with a Hach 2100Q portable turbidimeter at 25°C). The portable turbidimeter uses the nephelometric principle for measuring turbidity. The nephelometric turbidity measurement depends on the detection of the light scattered by the particles in suspension in the liquid. The measuring unit is the NTU. A 60 × 25 cm round borosilicate glass tank with a screw stopper is used. The amount of sample required is 15 mL. The measuring range is 0-1000 NTU. The samples are measured undiluted.

The cosmetic composition according to the present invention may preferably have a turbidity of between 1 and 200 NTU and preferably between 5 and 100 NTU.

### [Process and use]

The cosmetic composition according to the present invention may be used for a non-therapeutic process, such as a cosmetic process, for treating the skin, the hair, mucous membranes, the nails, the eyelashes, the eyelids and/or the scalp, by application to the skin, the hair, mucous membranes, the nails, the eyelashes, the eyelids or the scalp.

The present invention also relates to a use of the cosmetic composition according to the present invention, in its native form or in care products and/or washing products and/or makeup products and/or makeup-removing products for bodily and/or facial skin and/or mucous membranes and/or the scalp and/or the hair and/or the nails and/or the eyelashes and/or the eyelids.

The care product may be a lotion, a cream, a hair tonic, a hair conditioner, a sunscreen, and the like. The cleansing product may be a shampoo, a facial cleanser, a hand cleanser, and the like. The makeup product may be a foundation, a mascara, a lipstick, a lip gloss, a face powder, an eyeshadow, a nail varnish, and the like. The makeup-removing product may be a makeup-cleansing product, and the like.

### EXAMPLES

The present invention is described in greater detail by means of examples, which should not, however, be considered as limiting the scope of the present invention.

(Particle size) The particle size is measured using a Vasco-2 machine (Cordouan Technologies) under undiluted conditions.

(Transparency) The transparency is measured using a Hach 2100Q portable turbidimeter at 25°C. A 60 × 25 cm round borosilicate glass tank with a screw stopper is used. The amount of sample required is 15 mL. The measuring range is 0-1000 NTU. The samples are measured undiluted.

### [Example 1 and Comparative Example 2]

The following compositions according to Example 1 and Comparative Example 2, described in Table 1, are prepared by mixing the components described in Table 1 as follows: (1) mixing isopropyl myristate and polyglyceryl-5 laurate to form an oily phase; (2) heating the oily phase to about 75°C; (3) mixing water and (3R,4S,5R)-2-[2-hydroxy-4-(4-hydroxy-3-methoxyphenyl)butyl]tetrahydro-2H-pyran-3,4,5-triol containing 42.5% active material in propanediol and water; and (4) adding the aqueous phase to the oily phase with stirring to obtain an O/W emulsion.

The numerical values for the amounts of the components described in Table 1 are all based on weight percentages of active materials.

| | 1 | Comparative 2 |
|---|---|---|
| Isopropyl myristate | 12 | 12 |
| Octyldodecanol | 1 | 1 |
| Polyglyceryl-2 caprate (Sunsoft Q-10D-C from Taiyo Kagaku) | 4 | 4 |
| Polyglyceryl-5 laurate (Sunsoft A-121 E-C from Taiyo Kagaku) | 9 | 9 |
| (3R,4S,5R)-2-[2-hydroxy-4-(4-hydroxy-3-met hoxyphenyl)butyl]tetrahydro-2H-pyran-3,4,5-triol containing 42.5% active material in propanediol and water | 12.3% AM | - |
| Butylene glycol | 2 | 2 |
| Phenoxyethanol | 0,5 | 0,5 |
| Caprylyl glycol | 0.5 | 0,5 |
| Water qs | 100 | 100 |

The appearance, the oil particle size and the transparency of the O/W emulsions obtained according to Example 1 and Comparative Example 1 are described in Table 2.

### [Example 3 and Comparative Example 4]

| | 3 | Comparative 4 |
|---|---|---|
| Ethylhexyl palmitate | 5 | 5 |
| Polyglyceryl-5 laurate (Sunsoft A-121 E-C from Taiyo Kagaku) | 5 | 5 |
| Butylene glycol | 20 | 20 |
| Sodium methyl stearoyl taurate (Nikkol SMT from Nikko) | 0.1 | 0,1 |
| Phenoxyethanol | 0,5 | 0,5 |
| Caprylyl glycol | 0.5 | 0,5 |
| Hydroxypropyltetrahydropyrantriol | 1% AM | |
| Water qs | 100 | 100 |

As clearly indicated by the results below, it was observed that the cosmetic compositions in the form of an O/W emulsion according to the present invention 1 and 3 show better transparency and are stable.

**Table 2**

| | Macroscopic appearance | Mean turbidity (NTU) | Numerical mean diameter (nm) | Stability |
|---|---|---|---|---|
| 1 invention | Transparent fluid | 25 | 20 | Stable 2 months at 45°C |
| 2 Comparative | Opaque white fluid: 2 phases | > 1000 | > 1000 | 2 phases |
| 3 Invention | Transparent fluid | 77 | 30 | Stable 2 months at 45°C |
| 4 Comparative | Opaque white fluid: 2 phases | >1000 | > 1000 | 2 phases |

## Claims

1. Cosmetic composition in the form of a nanoemulsion or microemulsion, comprising:
(a) at least one oil;
(b) at least one nonionic surfactant with an HLB value from 8.0 to 14.0, preferably from 9.0 to 13.5 and preferably from 10.0 to 13.0;
(c) at least one C-glycoside compound;
(d) water;
the said non-ionic surfactant (b) is chosen from:
the surfactants that are fluid at a temperature of less than or equal to 45°C, chosen from esters of polyglycerols comprising from 2 to 12 glycerol units, and of at least one fatty acid comprising at least one saturated or unsaturated, linear or branched C₈-C₂₂ alkyl chain.

2. Cosmetic composition according to Claim 1, in which the oil (a) is chosen from the group consisting of oils of plant origin, mineral oils, synthetic oils, silicone oils and hydrocarbon-based oils.

3. Cosmetic composition according to Claim 1 or 2, in which the oil (a) is chosen from hydrocarbon-based oils which are in the form of a liquid at room temperature.

4. Cosmetic composition according to any one of Claims 1 to 3, in which the oil (a) is chosen from oils with a molecular weight of less than 600 g/mol.

5. Cosmetic composition according to any one of Claims 1 to 4, in which the amount of oil (a) is in the range from 0.1 % to 50% by weight, preferably from 1 % to 40% by weight and more preferably from 3% to 30% by weight relative to the total weight of the composition.

6. Cosmetic composition according to any one of Claims 1 to 5, **characterized in that** the nonionic surfactant (b) is chosen from:
- polyglyceryl monolaurate or dilaurate comprising 3 to 6 glycerol units,
- polyglyceryl mono(iso)stearate comprising 3 to 6 glycerol units,
- polyglyceryl monooleate comprising 3 to 6 glycerol units, and
- polyglyceryl dioleate comprising 3 to 6 glycerol units.

7. Cosmetic composition according to any one of Claims 1 to 6, in which the nonionic surfactant (b) is chosen from polyglyceryl fatty acid esters, preferably esters of a fatty acid and of polyglycerol comprising 70% by weight or more of polyglycerol in which the degree of polymerization is 4 or more, preferably esters of a fatty acid and of polyglycerol containing an amount greater than or equal to 60% by weight of polyglycerol in which the degree of polymerization is between 4 and 11, and more preferably esters of a fatty acid and of polyglycerol containing an amount greater than or equal to 30% by weight polyglycerol in which the degree of polymerization is 5.

8. Cosmetic composition according to any one of Claims 1 to 5, in which the amount of nonionic surfactant (b) is in the range from 0.1% to 30% by weight, preferably from 1% to 25% by weight and more preferably from 3% to 20% by weight relative to the total weight of the composition.

9. Cosmetic composition according to any one of Claims 1 to 8, in which the weight ratio of the nonionic surfactant (b) to the oil (a) is from 0.25 to 6, preferably from 0.3 to 3 and more preferably from 0.4 to 1.5.

10. Cosmetic composition according to any one of Claims 1 to 9, in which the C-glycoside compound (c) is represented by the following formula: 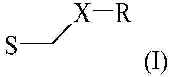 in which:
- R represents a saturated or unsaturated C₁-C₁₀ and in particular C₁-C₄ alkyl radical, which may be optionally substituted with at least one radical chosen from OH, COOH, Y and COOR"₂ with R"₂ being a saturated C₁-C₄ alkyl radical, Y denotes a phenyl radical or a heterocycle, optionally substituted with 1 to 5 (ORₐ) groups,
- S represents a monosaccharide or a polysaccharide comprising up to 20 sugar units, in particular up to 6 sugar units, in pyranose and/or furanose form and of the L and/or D series, it being possible for said monosaccharide or polysaccharide to be substituted with a hydroxyl group which must be free, and optionally with one or more optionally protected amine functions, and
- X represents a radical chosen from the following groups: -CO-, -CH(OR')-, -CH (NH₂)-, CHNR_{b}R_{c}; CHNHOR_{d}, -C(OR')-, -C (NH₂)-, CNR_{b}R_{c}; CNHOR_{d} -CH(NHCH₂CH₂CH₂OH)-, -CH(NHPh)- and -CH(CH₃)- and in particular a radical -CO-, -CH(OH)- or -CH(NH₂)- and more particularly a radical -CH(OH)-,
- R' denotes:
* a hydrogen atom;
* a saturated linear C1-C18 alkyl radical,
* an unsaturated linear C2-C18 alkyl radical,
* a saturated or unsaturated branched C3-C18 alkyl radical,
* a saturated or unsaturated C5 or C6 cyclic radical,
* a linear or branched, saturated or unsaturated C2-C18, or saturated or unsaturated C5 or C6 cyclic acyl radical.
**Rₐ** denotes:
- a hydrogen atom
- a linear or branched C1-C4 alkyl radical, or a linear or branched unsaturated C3-C4 hydrocarbon-based radical
- a linear or branched C2-C18 acyl or linear or branched C2-C18 alkenylcarbonyl radical
**R_{b}** denotes:
- a hydrogen atom
- a linear C2-C18 or branched C3-C18 alkyl radical, or a linear or branched unsaturated C3-C4 hydrocarbon-based radical or a radical -CH(Z₁)-CO₂Z₂ in which Z₁ denotes a hydrogen atom or a linear or branched C1-C6, or saturated or unsaturated cyclic C3-C6 alkyl radical, the said radical being optionally substituted with at least one group chosen from =NH, -NH₂, -N(T)₂, =O, -OH, -OT, -SH, -ST, -CO₂T, phenyl, phenyl substituted with -OH or -OT, 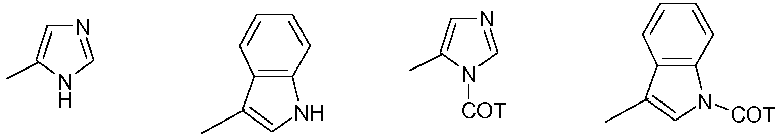 and/or interrupted with a group -NH-, -N-(COT)- or -S- with T denoting a linear or branched C1-C6 or cyclic C3-C6 alkyl radical.
and Z₂ denotes a hydrogen atom or a linear C1-C6 alkyl radical
**R_{c}** denotes:
- a hydrogen atom
- a linear C1-C4 or branched C3-C4 alkyl radical, or a linear or branched unsaturated C3-C4 hydrocarbon-based radical, the said radical being optionally substituted with a phenyl group.
**Rd** denotes:
- a hydrogen atom
- a linear C1-C18 or branched C3-C18 alkyl radical, or a linear or branched unsaturated C3-C18 hydrocarbon-based radical, the said radical being optionally substituted with a phenyl group,
the bond S-CH₂-X represents a bond of C-anomeric nature, which may be α or β,
and also the cosmetically acceptable salts thereof, solvates thereof such as hydrates, and optical and geometrical isomers thereof.

11. Cosmetic composition according to any one of Claims 1 to 10, in which the C-glycoside compound (c) is represented by the following formula: 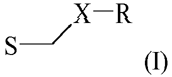 in which:
- R denotes an unsubstituted linear C₁-C₄ alkyl radical, especially C₁-C₂, in particular methyl;
- S represents a monosaccharide as described previously, chosen in particular from D-glucose, D-xylose, N-acetyl-D-glucosamine and L-fucose, and in particular D-xylose;
- X represents a group chosen from -CO-, -CH(OH)- and -CH(NH₂)- and preferentially a group -CH(OH)-.

12. Cosmetic composition according to any one of Claims 1 to 11, in which the C-glycoside compound (c) is C-β-D-xylopyranoside-2-hydroxypropane or C-α-D-xylopyranoside-2-hydroxypropane, and preferably C-β-D-xylopyranoside-2-hydroxypropane.

13. Cosmetic composition according to any one of Claims 1 to 10 in which the C-glycoside compound (c) is represented by formula (II) below: 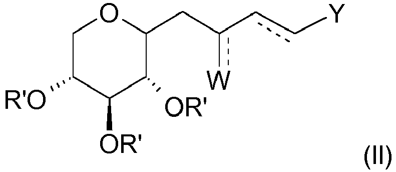 in which:
- the compounds of formula (II) are xylose derivatives
- Y denotes a phenyl radical or a heterocycle, optionally substituted with 1 to 5 groups (ORa)
- W = -OR'; (=O); NR_{b}Rc; NHOR_{d}
- R' denotes:
* a hydrogen atom;
* a saturated linear C1-C18 alkyl radical,
* an unsaturated linear C2-C18 alkyl radical,
* a saturated or unsaturated branched C3-C18 alkyl radical,
* a saturated or unsaturated C5 or C6 cyclic radical,
* a linear or branched, saturated or unsaturated C2-C18, or saturated or unsaturated C5 or C6 cyclic acyl radical.
**Rₐ** denotes:
- a hydrogen atom
- a linear or branched C1-C4 alkyl radical, or a linear or branched unsaturated C3-C4 hydrocarbon-based radical
- a linear or branched C2-C18 acyl or linear or branched C2-C18 alkenylcarbonyl radical
- when Y denotes a phenyl radical or a heterocycle substituted with 2 to 5 groups (ORₐ), two adjacent groups ORₐ may together form a divalent radical -O-CH₂-O with the proviso that when W = OH, the compound does not comprise an ethylenic double bond alpha to the carbon bearing this OH
**R_{b}** denotes:
- a hydrogen atom
- a linear C2-C18 or branched C3-C18 alkyl radical, or a linear or branched unsaturated C3-C4 hydrocarbon-based radical or a radical -CH(Z₁)-CO₂Z₂ in which Z₁ denotes a hydrogen atom or a linear or branched C1-C6, or saturated or unsaturated cyclic C3-C6 alkyl radical, the said radical being optionally substituted with at least one group chosen from =NH, -NH₂, -N(T)₂, =O, -OH, -OT, -SH, -ST, -CO₂T, phenyl, phenyl substituted with -OH or -OT, 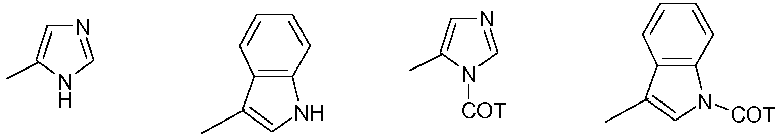 and/or interrupted with a group -NH-, -N-(COT)- or -S- with T denoting a linear or branched C1-C6 or cyclic C3-C6 alkyl radical.
and Z₂ denotes a hydrogen atom or a linear C1-C6 alkyl radical
**R_{c}** denotes:
- a hydrogen atom
- a linear C1-C4 or branched C3-C4 alkyl radical, or a linear or branched unsaturated C3-C4 hydrocarbon-based radical, the said radical being optionally substituted with a phenyl group.
**Rd** denotes:
- a hydrogen atom
- a linear C1-C18 or branched C3-C18 alkyl radical, or a linear or branched unsaturated C3-C18 hydrocarbon-based radical, the said radical being optionally substituted with a phenyl group,
and also the cosmetically acceptable salts thereof, solvates thereof such as hydrates, and stereoisomers thereof.

14. Composition according to any of claims 1 to 10 or 13 **characterized in that** the C-glycoside compound (c) is of formula (II) in which:
- the compounds of formula (II) are xylose derivatives
- Y denotes a phenyl radical or a heterocycle, optionally substituted with 1 to 3 groups (ORa)
- W = -OR'; (=O); NR_{b}Rc; NHOR_{d}
- R' denotes:
* a hydrogen atom,
* a linear or branched, saturated or unsaturated C1-C4 alkyl radical,
* a linear or branched C1-C6 acyl radical,
**Rₐ** denotes:
- a hydrogen atom
- a linear or branched C1-C4 alkyl radical,
- a linear or branched C1-C6 acyl radical,
when Y denotes a phenyl radical or a heterocycle substituted with 2 or 3 groups (ORₐ), two adjacent groups ORₐ may together form a divalent radical -O-CH₂-O with the proviso that when W = OH, the compound does not comprise an ethylenic double bond alpha to the carbon bearing this OH
**R_{b}** denotes:
- a hydrogen atom
- a linear C2-C8 or branched C3-C8 alkyl radical, or a radical -CH(Z₁)-CO₂Z₂ in which Z₁ denotes a hydrogen atom or a linear or branched C1-C6, or saturated or unsaturated cyclic C3-C6 alkyl radical, the said radical being optionally substituted with at least one group chosen from =NH, -NH₂, -N(T)₂, =O, -OH, -OT, -SH, -ST, -CO₂T, phenyl, phenyl substituted with -OH or -OT, 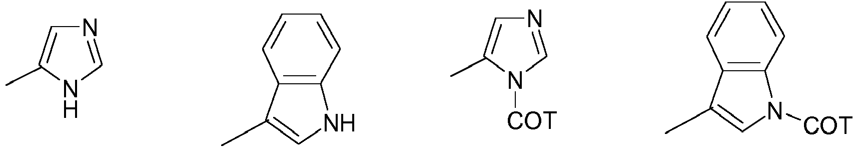 and/or interrupted with a group -NH-, -N-(COT)- or -S- with T denoting a linear C1-C6 or cyclic C5-C6 alkyl radical,
and Z₂ denotes a hydrogen atom or a linear C1-C6 alkyl radical
**R_{c}** denotes:
- a hydrogen atom
- a linear C1-C4 or branched C3-C4 alkyl radical, optionally substituted with a phenyl group,
**Rd** denotes:
- a hydrogen atom
- a linear C1-C4 or branched C3-C4 alkyl radical, the said radical being optionally substituted with a phenyl group,
and also the cosmetically acceptable salts thereof, solvates thereof such as hydrates, and stereoisomers thereof.

15. Composition according to any one claims 1 to 10 or 13 **characterized in that** the C-glycoside compound (c) is of formula (II) and is chosen from:
**Compound 1.** : (3R,4S,5R)-2-[2-hydroxy-4-(4-hydroxy-3-methoxyphenyl)butyl]tetrahydro-2H-pyran-3,4, 5-triol 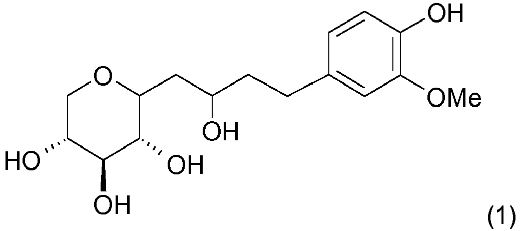
**Compound 2.** 4-(4-hydroxy-3-methoxyphenyl)-1-[(3R,4S,5R)-3,4,5-trihydroxytetrahydro-2H-pyran-2-yl butan-2-one 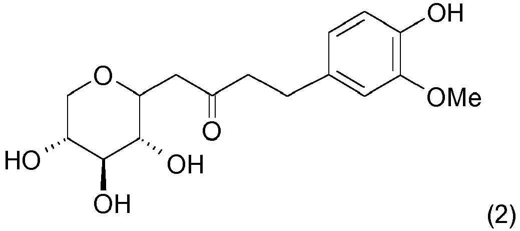
**Compound 3.** (3R,4S,5R)-2-[2-hydroxy-4-(4-hydroxyphenyl)butyl]tetrahydro-2H-pyran-3,4,5-triol 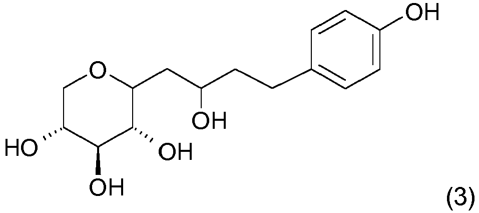
**Compound 4** 4-(4-hydroxyphenyl)-1-[(3R,4S,5R)-3,4,5-trihydroxytetrahydro-2H-pyran-2-yl]butan-2-on e 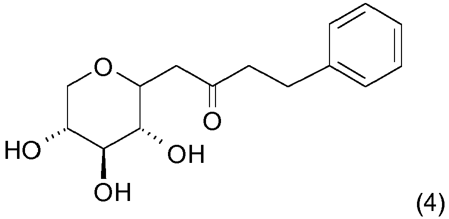
**Compound 5.** (3R,4S,5R)-2-[2-(benzylamino)-4-(4-hydroxy-3-methoxyphenyl)butyl]tetrahydro-2H-pyr an-3,4,5-triol 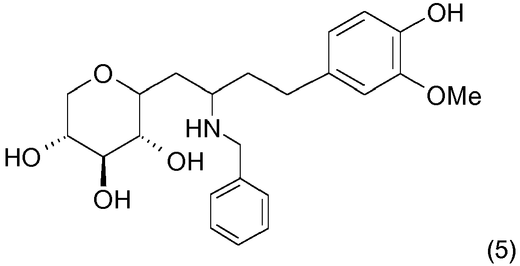
**Compound 6.** ethyl {[3-(4-hydroxy-3-methoxyphenyl)-1-{[(3R,4S,5R)-3,4,5-trihydroxytetra-hydro-2H-pyran-2-yl]methyl}propyl]amino}(phenyl)acetate 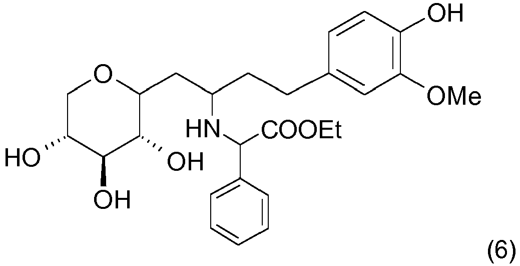
**Compound 7.** (3E)-4-phenyl-1-[(3R,4S,5R)-3,4,5-trihydroxytetrahydro-2H-pyran-2-yl]but-3-en-2-one 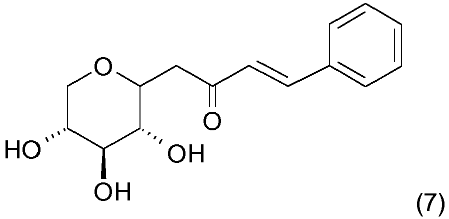
**Compound 8.** (3E)-4-(4-hydroxy-3,5-dimethoxyphenyl)-1-[(3R,4S,5R)-3,4,5-trihydroxytetrahydro-2H-p yran-2-yl]but-3-en-2-one 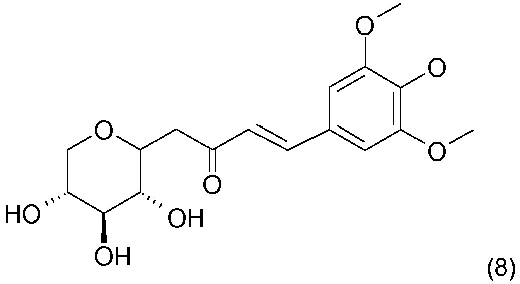
**Compound 9.** (3R,4S,5R)-2-[2-hydroxy-4-(2-hydroxyphenyl)butyl]tetrahydro-2H-pyran-3,4,5-triol 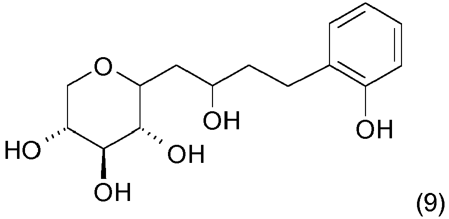
**Compound 10:** (3R,4S,5R)-2-[2-hydroxy-4-(3-hydroxy-4-methoxyphenyl)butyl]tetrahydro-2H-pyran-3,4, 5-triol 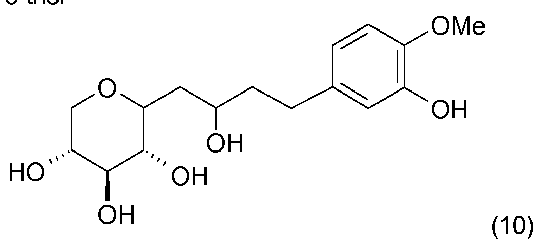
**Compound 11.** (3R,4S,5R)-2-[2-hydroxy-4-(2,4-di-hydroxyphenyl)butyl]tetrahydro-2H-pyran-3,4,5-triol 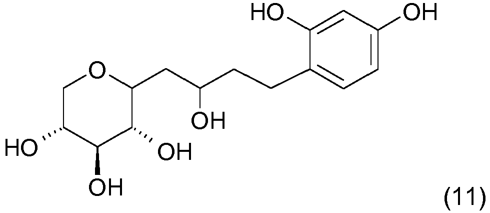
**Compound 12.** (3R,4S,5R)-2-[2-hydroxy-4-(3-ethoxy-4-hydroxyphenyl)butyl]tetrahydro-2H-pyran-3,4,5-triol 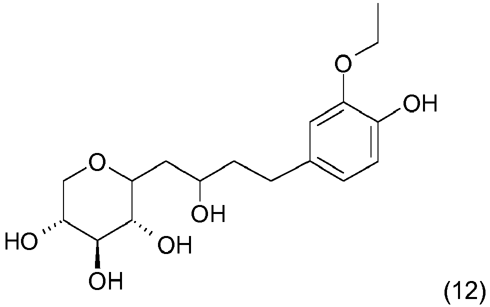
**Compound 13.** 5,9-anhydro-1,2,4-trideoxy-1-pyridin-3-yl-D-xylononitol 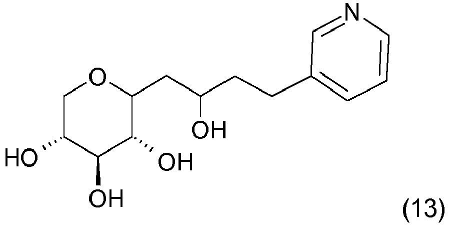
**Compound 14.** (3R,4S,5R)-2-[(2E)-2-(methoxyimino)-4-phenylbutyl]tetrahydro-2H-pyran-3,4,5-triol 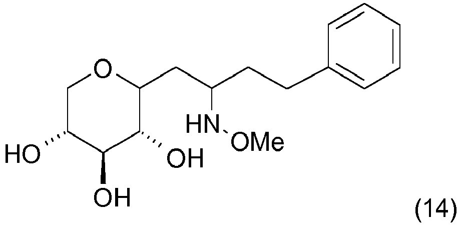
**Compound 15.** 5,9-anhydro-1,2,4-trideoxy-7-O-pentanoyl-1-phenyl-1-D-xylonon-3-ulose 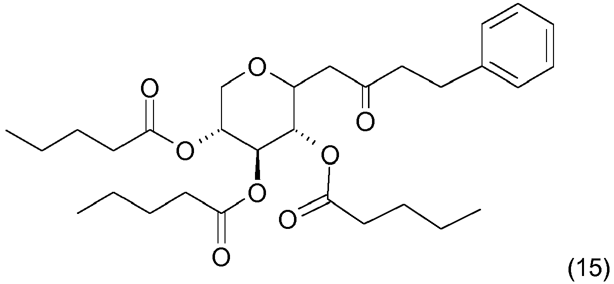
**Compound 16.** 5,9-anhydro-1,2,4-trideoxy-1-(3,4,5-trimethoxyphenyl)-D-xylononitol

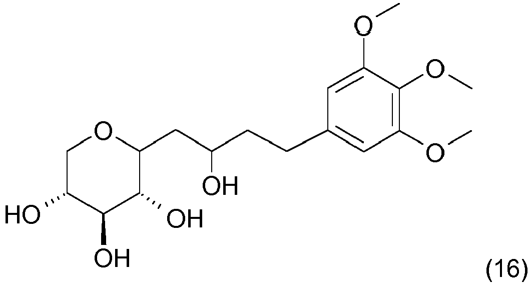

16. Composition according the preceding claim **characterized in that** the C-glycoside compound (c) is of formula (II) and is chosen from compounds (1) and (11).

17. Cosmetic composition according to any one of Claims 1 to 16, in which the amount of C-glycoside compound (c) is in the range from 0.01% to 20% by weight, preferably from 0.1% to 15% by weight and more preferably from 1% to 10% by weight relative to the total weight of the composition.

18. Cosmetic composition according to any one of Claims 1 to 17, also comprising at least one nonionic surfactant other than (b) above and/or at least one ionic surfactant.

19. Cosmetic composition according to any one of Claims 1 to 18, also comprising at least one polyol.

20. Cosmetic composition according to any one of Claims 1 to 19, in which the cosmetic composition is in the form of an O/W emulsion, and the oil (a) is in the form of droplets with a numerical mean particle size of 300 nm or less, preferably from 10 nm to 150 nm.

21. Non-therapeutic process for treating the skin, the hair, mucous membranes, the nails, the eyelashes, the eyelids and/or the scalp, **characterized in that** the cosmetic composition according to any one of Claims 1 to 20 is applied to the skin, the hair, mucous membranes, the nails, the eyelashes, the eyelids or the scalp.

22. Use of the cosmetic composition according to any one of Claims 1 to 20 as or in care products and/or washing products and/or makeup products and/or makeup-removing products for bodily and/or facial skin and/or mucous membranes and/or the scalp and/or the hair and/or the nails and/or the eyelashes and/or the eyelids.

## Patentansprüche

1. Kosmetische Zusammensetzung in Form einer Nanoemulsion oder Mikroemulsion, umfassend:
(a) mindestens ein Öl;
(b) mindestens ein nichtionisches Tensid mit einem HLB-Wert von 8,0 bis 14,0, vorzugsweise von 9,0 bis 13,5 und vorzugsweise von 10,0 bis 13,0;
(c) mindestens eine C-Glycosid-Verbindung;
(d) Wasser;
wobei das nichtionische Tensid (b) ausgewählt ist aus:
den Tensiden, die bei einer Temperatur kleiner oder gleich 45 °C fließfähig sind, ausgewählt aus Estern von Polyglycerinen mit 2 bis 12 Glycerin-Einheiten und mindestens einer Fettsäure mit mindestens einer gesättigten oder ungesättigten, linearen oder verzweigten C₈-C₂₂-Alkylkette.

2. Kosmetische Zusammensetzung nach Anspruch 1, wobei das Öl (a) aus der Gruppe besthend aus Ölen pflanzlichen Ursprungs, Mineralölen, synthetischen Ölen, Silikonölen und Ölen auf Kohlenwasserstoffbasis ausgewählt ist.

3. Kosmetische Zusammensetzung nach Anspruch 1 oder 2, wobei das Öl (a) aus Ölen auf Kohlenwasserstoffbasis, die bei Raumtemperatur in Form einer Flüssigkeit vorliegen, ausgewählt ist.

4. Kosmetische Zusammensetzung nach einem der Ansprüche 1 bis 3, wobei das Öl (a) aus Ölen mit einem Molekulargewicht von weniger als 600 g/mol ausgewählt ist.

5. Kosmetische Zusammensetzung nach einem der Ansprüche 1 bis 4, wobei die Menge von Öl (a) im Bereich von 0,1 bis 50 Gew.-%, vorzugsweise von 1 bis 40 Gew.-% und weiter bevorzugt von 3 bis 30 Gew.-%, bezogen auf das Gesamtgewicht der Zusammensetzung, liegt.

6. Kosmetische Zusammensetzung nach einem der Ansprüche 1 bis 5, **dadurch gekennzeichnet, dass** das nichtionische Tensid (b) ausgewählt ist aus:
- Polyglycerylmonolaurat oder -dilaurat mit 3 bis 6 Glycerin-Einheiten,
- Polyglycerylmono(iso)stearat mit 3 bis 6 Glycerin-Einheiten,
- Polyglycerylmonooleat mit 3 bis 6 Glycerin-Einheiten und
- Polyglyceryldioleat mit 3 bis 6 Glycerin-Einheiten.

7. Kosmetische Zusammensetzung nach einem der Ansprüche 1 bis 6, wobei das nichtionische Tensid (b) aus Polyglycerylfettsäureestern, vorzugsweise Estern von einer Fettsäure und Polyglycerin, die 70 Gew.-% oder mehr Polyglycerin mit einem Polymerisationsgrad von 4 oder mehr umfassen, vorzugsweise Estern von einer Fettsäure und Polyglycerin, die eine Menge größer oder gleich 60 Gew.-% Polyglycerin mit einem Polymerisationsgrad zwischen 4 und 11 enthalten, und weiter bevorzugt Estern von einer Fettsäure und Polyglycerin mit einer Menge größer oder gleich 30 Gew.-% Polyglycerin mit einem Polymerisationsgrad von 5 ausgewählt ist.

8. Kosmetische Zusammensetzung nach einem der Ansprüche 1 bis 5, wobei die Menge von nichtionischem Tensid (b) im Bereich von 0,1 bis 30 Gew.-%, vorzugsweise 1 bis 25 Gew.-% und weiter bevorzugt 3 bis 20 Gew.-%, bezogen auf das Gesamtgewicht der Zusammensetzung, liegt.

9. Kosmetische Zusammensetzung nach einem der Ansprüche 1 bis 8, wobei das Gewichtsverhältnis von nichtionischem Tensid (b) zu Öl (a) 0,25 bis 6, vorzugsweise 0,3 bis 3 und weiter bevorzugt 0,4 bis 1,5 beträgt.

10. Kosmetische Zusammensetzung nach einem der Ansprüche 1 bis 9, wobei die C-Glycosid-Verbindung (c) durch die folgende Formel wiedergegeben wird: 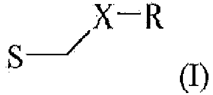 wobei:
- R für einen gesättigten oder ungesättigten C₁-C₁₀- und insbesondere C₁-C₄-Alkylrest, der gegebenenfalls durch mindestens einen Rest, der aus OH, COOH, Y und COOR''₂ ausgewählt ist, substituiert sein kann, steht, wobei R''₂ für einen gesättigten C₁-C₄-Alkylrest steht und Y für einen Phenylrest oder einen Heterocyclus, die gegebenenfalls durch 1 bis 5 (ORₐ) -Gruppen substituiert sind, steht,
- S für ein Monosaccharid oder ein Polysaccharid mit bis zu 20 Zuckereinheiten, insbesondere bis zu 6 Zuckereinheiten, in Pyranose- und/oder Furanose-Form und der L- und/oder D-Reihe steht, wobei das Monosaccharid oder Polysaccharid durch eine Hydroxylgruppe, die frei sein muss, und gegebenenfalls durch eine oder mehrere gegebenenfalls geschützte Aminfunktionen substituiert sein kann, und
- X für einen Rest, der aus den Gruppen -CO-, -CH(OR'), -CH(NH₂)-, CHNR_{b}R_{c}; CHNHOR_{d}, -C(OR')-, -C(NH₂)-, CNR_{b}R_{c}; CNHOR_{d}, -CH(NHCH₂CH₂CH₂OH)-, -CH(NHPh)- und -CH(CH₃) ausgewählt ist und speziell einen Rest -CO-, -CH(OH)- oder -CH(NH₂)- und spezieller einen Rest -CH(OH)- steht,
- R' für:
* ein Wasserstoffatom,
* einen gesättigten linearen C1-C18-Alkylrest,
* einen ungesättigten linearen C2-C18-Alkylrest,
* einen gesättigten oder ungesättigten verzweigten C3-C18-Alkylrest,
* einen gesättigten oder ungesättigten cyclischen C5- oder C6-Rest,
* einen linearen oder verzweigten, gesättigten oder ungesättigten C2-C18-Acylrest oder einen gesättigten oder ungesättigten cyclischen C5- oder C6-Acylrest steht,
**Rₐ** für:
- ein Wasserstoffatom,
- einen linearen oder verzweigten C1-C4-Alkylrest oder einen linearen oder verzweigten ungesättigten C3-C4-Kohlenwasserstoffrest,
- einen linearen oder verzweigten C2-C18-Acylrest oder einen linearen oder verzweigten C2-C18-Alkenylcarbonylrest
steht,
**R_{b}** für :
- ein Wasserstoffatom,
- einen linearen C2-C18- oder verzweigten C3-C18-Alkylrest oder einen linearen oder verzweigten ungesättigten C3-C4-Kohlenwasserstoffrest oder einen Rest -CH(Z₁)-CO₂Z₂, in dem Z₁ für ein Wasserstoffatom oder einen linearen oder verzweigten C1-C6-Alkylrest oder einen gesättigten oder ungesättigten cyclischen C3-C6-Alkylrest steht, wobei der Rest gegebenenfalls durch mindestens eine Gruppe, die aus =NH, -NH₂, -N(T)₂, =O, -OH, -OT, -SH, -ST, -CO₂T, Phenyl, Phenyl, das durch -OH oder -OT substituiert ist, 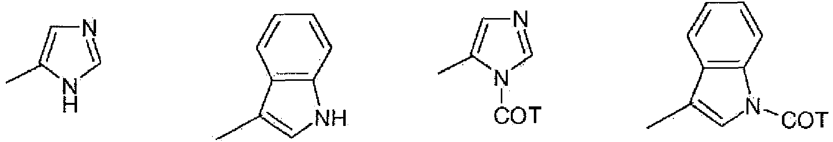 ausgewählt ist, substituiert und/oder durch eine Gruppe -NH-, -N-(COT)- oder -S- unterbrochen ist, wobei T für einen linearen oder verzweigten C1-C6-Alkylrest oder einen cyclischen C3-C6-Alkylrest steht,
und Z₂ für ein Wasserstoffatom oder einen linearen C1-C6-Alkylrest steht,
steht,
**R_{c}** für:
- ein Wasserstoffatom,
- einen linearen C1-C4- oder verzweigten C3-C4-Alkylrest oder einen linearen oder verzweigten ungesättigten C3-C4-Kohlenwasserstoffrest, wobei der Rest gegebenenfalls durch eine Phenylgruppe substituiert ist,
steht,
**R_{d}** für:
- ein Wasserstoffatom,
- einen linearen C1-C18- oder verzweigten C3-C18-Alkylrest oder einen linearen oder verzweigten ungesättigten C3-C18-Kohlenwasserstoffrest, wobei der Rest gegebenenfalls durch eine Phenylgruppe substituiert ist,
steht,
die Bindung S-CH₂-X für eine Bindung C-anomerer Natur, die α oder β sein kann, steht,
sowie die kosmetisch unbedenklichen Salze davon, Solvate davon wie Hydrate und optische und geometrische Isomere davon.

11. Kosmetische Zusammensetzung nach einem der Ansprüche 1 bis 10, wobei die C-Glycosid-Verbindung (c) durch die folgende Formel wiedergegeben wird: 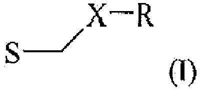 wobei:
- R für einen unsubstituierten linearen C₁-C₄-Alkylrest, insbesondere C₁-C₂-Alkylrest, speziell Methyl, steht,
- S für ein Monosaccharid gemäß obiger Beschreibung, das insbesondere aus D-Glucose, D-Xylose, N-Acetyl-D-glucösamin und L-Fucose ausgewählt ist, und insbesondere D-Xylose steht;
- X für eine Gruppe, die aus -CO-, -CH(OH)- oder -CH(NH₂)- ausgewählt ist, und vorzugsweise eine Gruppe -CH(OH)- steht.

12. Kosmetische Zusammensetzung nach einem der Ansprüche 1 bis 11, wobei es sich bei der C-Glycosid-Verbindung (c) um C-β-D-Xylopyranosid-2-hydroxypropan oder C-α-D-Xylopyranosid-2-hydroxypropan und vorzugsweise C-β-D-Xylopyranosid-2-hydroxypropan handelt.

13. Kosmetische Zusammensetzung nach einem der Ansprüche 1 bis 10, wobei die C-Glycosid-Verbindung (c) durch die nachstehende Formel (II) wiedergegeben wird; 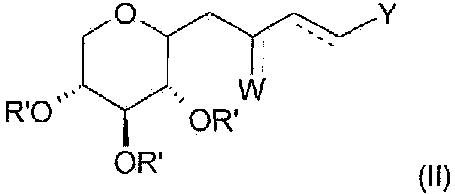 wobei:
- es sich bei den Verbindungen der Formel (II) um Xylosederivate handelt,
- Y für einen Phenylrest oder einen Heterocyclus, die gegebenenfalls durch 1 bis 5 Gruppen (ORₐ) substituiert sind, steht,
- W = -OR¹; (=O); NR_{b}R_{c}; NHOR_{d},
- R' für:
* ein Wasserstoffatom,
* einen gesättigten linearen C1-C18-Alkylrest,
* einen ungesättigten linearen C2-C18-Alkylrest,
* einen gesättigten oder ungesättigten verzweigten C3-C18-Alkylrest,
* einen gesättigten oder ungesättigten cyclischen C5- oder C6-Rest,
* einen linearen oder verzweigten, gesättigtten oder ungesättigten C2-C18-Acylrest oder einen gesättigten oder ungesättigten cyclischen C5- oder C6-Acylrest steht,
**Rₐ** für:
- ein Wasserstoffatom,
- einen linearen oder verzweigten C1-C4-Alkylrest oder einen linearen oder verzweigten ungesättigten C3-C4-Kohlenwasserstoffrest,
- einen linearen oder verzweigten C2-C18-Acylrest oder einen linearen oder verzweigten C2-C18-Alkenylcarbonylrest
steht,
dann, wenn Y für einen Phenylrest oder einen Heterocyclus, der durch 2 bis 5 Gruppen (ORₐ) substituiert ist, steht, zwei benachbarte Gruppen ORₐ zusammen einen zweiwertigen Rest -O-CH₂-O bilden können,
mit der Maßgabe, dass im Fall von W = OH die Verbindung keine ethylenische Doppelbindung in alpha-Position zu dem dieses OH tragenden Kohlenstoff enthält,
**R_{b}** für:
- ein Wasserstoffatom,
- einen linearen C2-C18- oder verzweigten C3-C18-Alkylrest oder einen linearen oder verzweigten ungesättigten C3-C4-Kohlenwasserstoffrest oder einen Rest -CH(Z₁)-CO₂Z₂, in dem Z₁ für ein Wasserstoffatom oder einen linearen oder verzweigten C1-C6-Alkylrest oder einen gesättigten oder ungesättigten cyclischen C3-C6-Alkylrest steht, wobei der Rest gegebenenfalls durch mindestens eine Gruppe, die aus =NH, -NH₂, -N(T)₂, =O, -OH, -OT, -SH, -ST, -CO₂T, Phenyl, Phenyl, das durch -OH oder -OT substituiert ist, 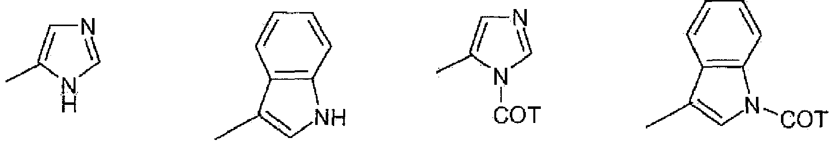 ausgewählt ist, substituiert und/oder durch eine Gruppe -NH-, -N-(COT)- oder -S- unterbrochen ist, wobei T für einen linearen oder verzweigten C1-C6-Alkylrest oder einen cyclischen C3-C6-Alkylrest steht,
und Z₂ für ein Wasserstoffatom oder einen linearen C1-C6-Alkylrest steht,
steht,
**R_{c}** für:
- ein Wasserstoffatom,
- einen linearen C1-C4- oder verzweigten C3-C4-Alkylrest oder einen linearen oder verzweigten ungesättigten C3-C4-Kohlenwasserstoffrest, wobei der Rest gegebenenfalls durch eine Phenylgruppe substituiert ist,
steht,
**R_{d}** für:
- ein Wasserstoffatom,
- einen linearen C1-C18- oder verzweigten C3-C18-Alkylrest oder einen linearen oder verzweigten ungesättigten C3-C18-Kohlenwasserstoffrest, wobei der Rest gegebenenfalls durch eine Phenylgruppe substituiert ist,
steht,
sowie die kosmetisch unbedenklichen Salze davon, Solvate davon wie Hydrate und Stereoisomere davon.

14. Zusammensetzung nach einem der Ansprüche 1 bis 10 oder 13, **dadurch gekennzeichnet, dass** die C-Glycosid-Verbindung (c) die Formel (II) aufweist, wobei:
- es sich bei den Verbindungen der Formel (II) um Xylosederivate handelt,
- Y für einen Phenylrest oder einen Heterocyclus, die gegebenenfalls durch 1 bis 3 Gruppen (ORₐ) substituiert sind, steht,
- W = -OR'; (=O); NR_{b}R_{c}; NHOR_{d},
- R' für:
* ein wasserstoffatom,
* einen linearen oder verzweigten, gesättigten oder ungesättigten C1-C4-Alkylrest,
* einen linearen oder verzweigten C1-C6-Acylrest
steht,
**Rₐ** für:
- ein Wasserstoffatom,
- einen linearen oder verzweigten C1-C4-Alkylrest,
- einen linearen oder verzweigten C1-C6-Acylrest steht,
dann, wenn Y für einen Phenylrest oder einen Heterocyclus, der durch 2 oder 3 Gruppen (ORₐ) substituiert ist, steht, zwei benachbarte Gruppen ORₐ zusammen einen zweiwertigen Rest -O-CH₂-O bilden können,
mit der Maßgabe, dass im Fall von W = OH die Verbindung keine ethylenische Doppelbindung in alpha-Position zu dem dieses OH tragenden Kohlenstoff enthält,
**R_{b}** für:
- ein Wasserstoffatom,
- einen linearen C2-C8- oder verzweigten C3-C8-Alkylrest oder einen Rest -CH(Z₁)-CO₂Z₂, in dem Z₁ für ein Wasserstoffatom oder einen linearen oder verzweigten C1-C6-Alkylrest oder einen gesättigten oder ungesättigten cyclischen C3-C6-Alkylrest steht, wobei der Rest gegebenenfalls durch mindestens eine Gruppe, die aus =NH, -NH₂, -N(T)₂, =O, -OH, -OT, -SH, -ST, -CO₂T, Phenyl, Phenyl, das durch -OH oder -OT substituiert ist, 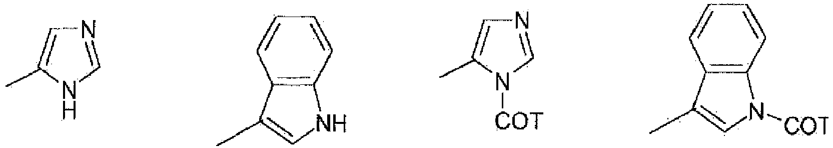 ausgewählt ist, substituiert und/oder durch eine Gruppe -NH-, -N-(COT)- oder -S- unterbrochen ist, wobei T für einen linearen C1-C6-Alkylrest oder einen cyclischen C5-C6-Alkylrest steht,
und Z₂ für ein Wasserstoffatom oder einen linearen C1-C6-Alkylrest steht,
steht,
**R_{c}** für:
- ein Wasserstoffatom,
- einen linearen C1-C4- oder verzweigten C3-C4-Alkylrest, der gegebenenfalls durch eine Phenylgruppe substituiert ist,
steht,
**R_{d}** für:
- ein Wasserstoffatom,
- einen linearen C1-C4- oder verzweigten C3-C4-Alkylrest, wobei der Rest gegebenenfalls durch eine Phenylgruppe substituiert ist,
steht,
sowie die kosmetisch unbedenklichen Salze davon, Solvate davon wie Hydrate und Stereoisomere davon.

15. Zusammensetzung nach einem der Ansprüche 1 bis 10 oder 13, **dadurch gekennzeichnet, dass** die C-Glycosid-Verbindung (c) die Formel (II) aufweist und ausgewählt ist aus:
**Verbindung 1.** (3R,4S,5R)-2-[2-Hydroxy-4-(4-hydroxy-3-methoxyphenyl)butyl]tetrahydro-2H-pyran-3,4,5-triol 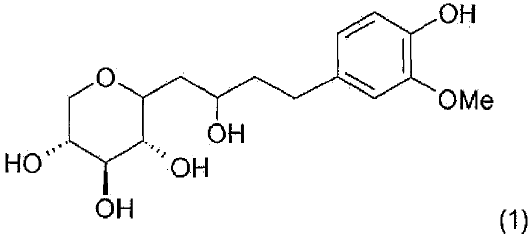
**Verbindung 2.** 4-(4-Hydroxy-3-methoxyphenyl)-1-[(3R,4S,5R)-3,4,5-trihydroxytetrahydro-2H-pyran-2-yl]butan-2-on 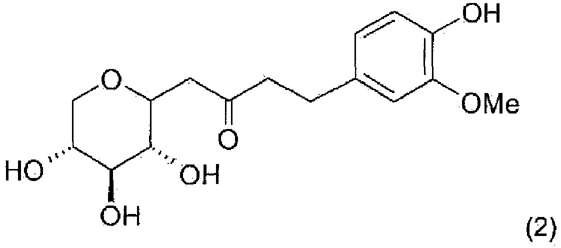
**Verbindung 3.** (3R,4S,5R)-2-[2-Hydroxy-4-(4-hydroxyphenyl)butyl]-tetrahydro-2H-pyran-3,4,5-triol 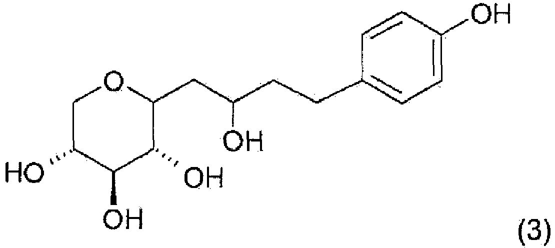
**Verbindung 4.** 4-(4-Hydroxyphenyl)-1-[(3R,4S,5R)-3,4,5-trihydroxytetrahydro-2H-pyran-2-yl]butan-2-on 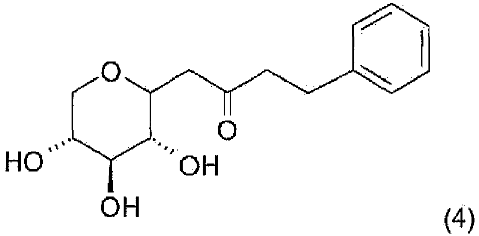
**Verbindung 5.** (3R,4S,5R)-2-[2-(Benzylamino)-4-(4-hydroxy-3-methoxyphenyl)butyl]tetrahydro-2H-pyran-3,4,5-triol 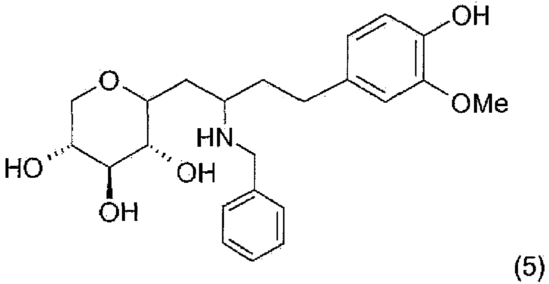
**Verbindung 6.** {[3-(4-Hydroxy-3-methoxyphenyl)-1-{[Z3R,4S,5R)-3,4,5-trihydroxytetrahydro-2H-pyran-2-yl]methyl}-propyl]amino}(phenyl)essigsäureethylester 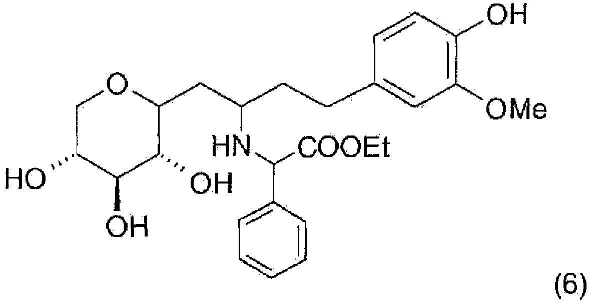
**Verbindung 7.** (3E)-4-Phenyl-1-[(3R,4S,5R)-3,4,5-trihydroxytetrahydro-2H-pyran-2-yl]but-3-en-2-on 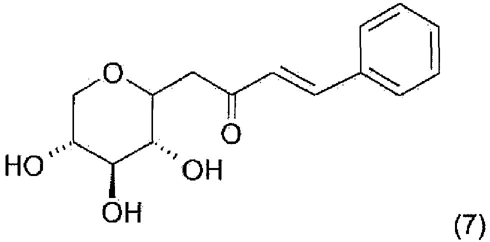
**Verbindung 8.** (3E)-4-(4-Hydroxy-3,5-dimethoxyphenyl)-1-[(3R,4S,5R)-3,4,5-trihydroxytetrahydro-2H-pyran-2-yl]but-3-en-2-on 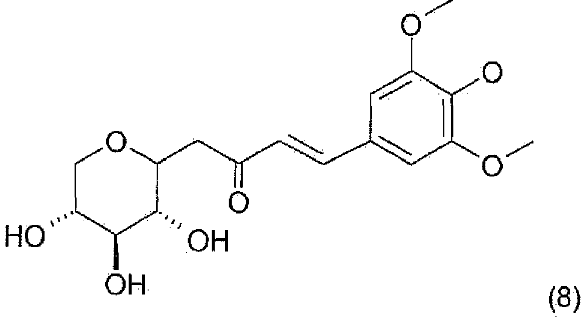
**Verbindung 9.** (3R,4S,5R)-2-[2-Hydroxy-4-(2-hydroxyphenyl)butyl]-tetrahydro-2H-pyran-3,4,5-triol 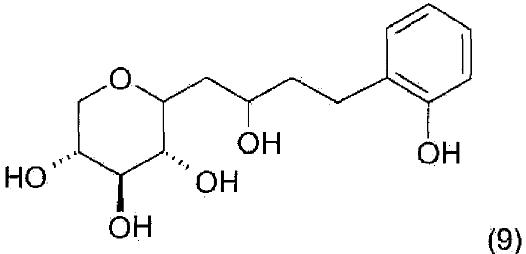
**Verbindung 10:** (3R,4S,5R)-2-[2-Hydroxy-4-(3-hydroxy-4-methoxyphenyl)butyl]tetrahydro-2H-pyran-3,4,5-triol 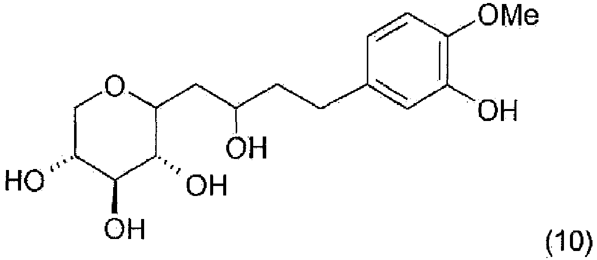
**Verbindung 11.** (3R,4S,5R)-2-[2-Hydroxy-4-(2,4-dihydroxyphenyl)butyl]tetrahydro-2H-pyran-3,4,5-triol 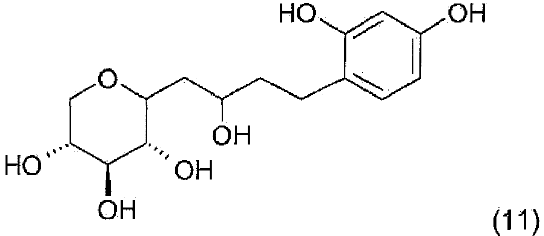
**Verbindung 12.** (3R,4S,5R)-2-[2-Hydroxy-4-(3-ethoxy-4-hydroxyphenyl)butyl]tetrahydro-2H-pyran-3,4,5-triol 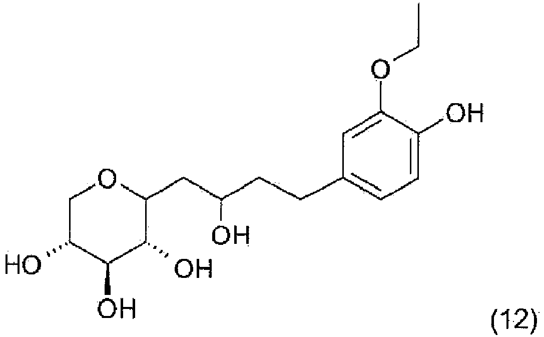
**Verbindung 13.** 5,9-Anhydro-1,2,4-tridesoxy-1-pyridin-3-yl-D-xylononitol 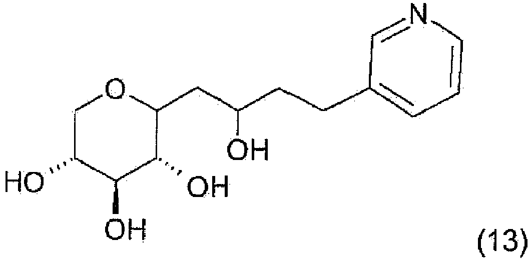
**Verbindung 14.** (3R,4S,5R)-2-[(2E)-2-(Methoxyimino)-4-phenylbutyl]tetrahydro-2H-pyran-3,4,5-triol 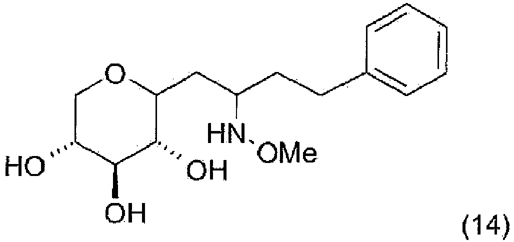
**Verbindung 15.** 5,9-Anhydro-1,2,4-tridesoxy-7-O-pentanoyl-1-phenyl-D-xylonon-3-ulose 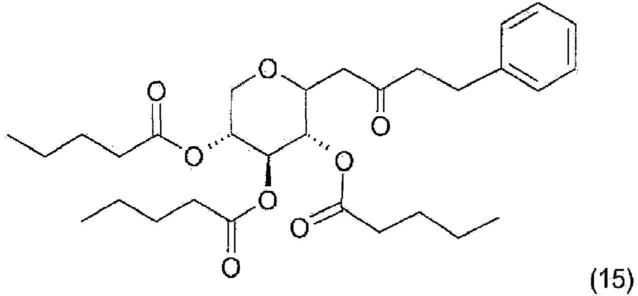
**Verbindung 16.** 5,9-Anhydro-1,2,4-tridesoxy-1-(3,4,5-trimethoxyphenyl)-D-xylononitol

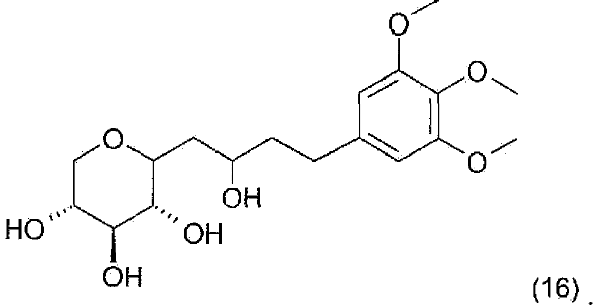

16. Zusammensetzung nach dem vorhergehenden Anspruch, **dadurch gekennzeichnet, dass** die C-Glycosid-Verbindung (c) die Formel (II) aufweist und aus den Verbindungen (1) und (11) ausgewählt ist.

17. Kosmetische Zusammensetzung nach einem der Ansprüche 1 bis 16, wobei die Menge von C-Glycosid-Verbindung (c) im Bereich von 0,01 bis 20 Gew.-%, vorzugsweise von 0,1 bis 15 Gew.-% und weiter bevorzugt von 1 bis 10 Gew.-%, bezogen auf das Gesamtgewicht der Zusammensetzung, liegt.

18. Kosmetische Zusammensetzung nach einem der Ansprüche 1 bis 17, die außerdem mindestens ein nichtionisches Tensid, das von (b) oben verschieden ist, und/oder mindestens ein ionisches Tensid umfasst.

19. Kosmetische Zusammensetzung nach einem der Ansprüche 1 bis 18, die außerdem mindestens ein Polyol umfasst.

20. Kosmetische Zusammensetzung nach einem der Ansprüche 1 bis 19, wobei die kosmetische Zusammensetzung in Form einer O/W-Emulsion vorliegt und das Öl (a) in Form von Tröpfchen mit einer zahlenmittleren Teilchengröße von 300 nm oder weniger, vorzugsweise von 10 nm bis 150 nm, vorliegt.

21. Nichttherapeutisches Verfahren zur Behandlung der Haut, des Haars, der Schleimhäute, der Nägel, der Wimpern, der Augenlider und/oder der Kopfhaut, **dadurch gekennzeichnet, dass** die kosmetische Zusammensetzung nach einem der Ansprüche 1 bis 20 auf die Haut, das Haar, die Schleimhäute, die Nägel, die Wimpern, die Augenlider oder die Kopfhaut aufgebracht wird.

22. Verwendung der kosmetischen Zusammensetzung nach einem der Ansprüche 1 bis 20 als oder in Pflegeprodukte/n und/oder Waschprodukte/n und/oder Schminkprodukte/n und/oder Abschminkprodukte/n für die Körper- und/oder Gesichtshaut und/oder Schleimhäute und/oder die Kopfhaut und/oder das Haar und/oder die Nägel und/oder die Wimpern und/oder die Augenlider.

## Revendications

1. Composition cosmétique sous la forme d'une nano- ou micro-émulsion, comprenant :
(a)au moins une huile ;
(b)au moins un tensioactif non ionique ayant une valeur HLB de 8,0 à 14,0, de préférence de 9,0 à 13,5, et de préférence de 10,0 à 13,0 ;
(c) au moins un composé C-glycoside ;
(d) de l'eau ;
Ledit tensioactif non ionique (b) étant choisi parmi :
des tensioactifs qui sont fluides à une température inférieure ou égale à 45 °C, choisis parmi les esters d'au moins un polyol choisi dans le groupe formé par un polyéthylèneglycol comprenant de 1 à 60 motifs d'oxyde d'éthylène, le sorbitane, le glycérol comprenant de 2 à 30 motifs d'oxyde d'éthylène, des polyglycérols comprenant de 2 à 12 motifs de glycérol, et d'au moins un acide gras comprenant au moins une chaîne alkyle en C₈-C₂₂ linéaire ou ramifiée, saturée ou insaturée,

2. Composition cosmétique selon la revendication 1, dans laquelle l'huile (a) est choisie dans le groupe constitué d'huiles d'origine végétale, d'huiles minérales, d'huiles synthétiques, d'huiles silicones et d'huiles hydrocarbonées.

3. Composition cosmétique selon la revendication 1 ou 2, dans laquelle l'huile (a) est choisie parmi des huiles hydrocarbonées qui sont sous la forme d'un liquide à température ambiante.

4. Composition cosmétique selon l'une quelconque des revendications 1 à 3, dans laquelle l'huile (a) est choisie parmi des huiles ayant un poids moléculaire inférieur à 600 g/mol.

5. Composition cosmétique selon l'une quelconque des revendications 1 à 4, dans laquelle la quantité de l'huile (a) est dans la plage de 0,1 à 50 % en poids, de préférence de 1 à 40 % en poids, et plus préférablement de 3 à 30 % en poids, par rapport au poids total de la composition.

6. Composition cosmétique selon l'une quelconque des revendications 1 à 5, dans laquelle le tensioactif non ionique (b) est choisi parmi :
- le monolaurate ou dilaurate de polyglycéryle comprenant 3 à 6 motifs de glycérol,
- le mono(iso)stéarate de polyglycéryle comprenant 3 à 6 motifs de glycérol,
- le monooléate de polyglycéryle comprenant 3 à 6 motifs de glycérol, et
- le dioléate de polyglycéryle comprenant 3 à 6 motifs de glycérol.

7. Composition cosmétique selon l'une quelconque des revendications 1 à 6, dans laquelle le tensioactif non ionique (b) est choisi parmi des esters d'acide gras de polyglycéryle, de préférence des esters d'un acide gras et de polyglycérol comprenant 70 % en poids ou plus de polyglycérol dont le degré de polymérisation est 4 ou plus, de préférence des esters d'un acide gras et de polyglycérol contenant une quantité égale ou supérieure à 60 % en poids de polyglycérol dont le degré de polymérisation est compris entre 4 et 11, et plus préférablement des esters d'un acide gras et de polyglycérol contenant une quantité égale ou supérieure à 30 % en poids de polyglycérol dont le degré de polymérisation est 5.

8. Composition cosmétique selon l'une quelconque des revendications 1 à 5, dans laquelle la quantité du tensioactif non ionique (b) est dans la plage de 0,1 à 30 % en poids, de préférence de 1 à 25 % en poids, et plus préférablement de 3 à 20 % en poids, par rapport au poids total de la composition.

9. Composition cosmétique selon l'une quelconque des revendications 1 à 8, dans laquelle le rapport en poids du tensioactif non ionique (b) à l'huile (a) est de 0,25 à 6, de préférence de 0,3 à 3, et plus préférablement de 0,4 à 1,5.

10. Composition cosmétique selon l'une quelconque des revendications 1 à 19, dans laquelle le composé C-glycoside (c) est représenté par la formule suivante : 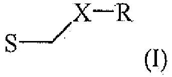 dans laquelle :
- R représente un radical alkyle, saturé ou insaturé en C₁ à C₁₀, en particulier en C₁ à C₄, et pouvant être éventuellement substitué par au moins un radical choisi parmi OH, COOH, Y ou COOR"₂, avec R"₂ étant un radical alkyle en C₁-C₄ saturé, Y désigne un radical phényle ou un hétérocycle, éventuellement substitués par 1 à 5 groupements (ORₐ),
- S représente un monosaccharide ou un polysaccharide comportant jusqu'à 20 unités sucre, en particulier jusqu'à 6 unités sucre, sous forme pyranose et/ou furanose et de série L et/ou D, ledit mono- ou polysaccharide pouvant être substitué par un groupement hydroxyle obligatoirement libre, et éventuellement une ou plusieurs fonction(s) amine(s) éventuellement protégée(s), et
- X représente un radical choisi parmi les groupements -CO-, -CH(OR')-, -CH(NH₂)-, CHNR_{b}R_{c} ; CHNHOR_{d},-C(OR')-, -C(NH₂)-, CNR_{b}R_{c} ; CNHOR_{d}-CH(NHCH₂CH₂CH₂OH)-, -CH(NHPh)- et -CH(CH₃)- et en particulier un radical-CO-, -CH(OH)- ou -CH(NH₂)- et plus particulièrement un radical -CH(OH)-,
- R' désigne :
* un atome d'hydrogène,
* un radical alkyle linéaire et saturé en C1-C18,
*un radical alkyle linéaire et insaturé C2-C18,
* un radical alkyle ramifié, saturé ou insaturé, en C3-C18,
* un radical cyclique saturé ou insaturé en C5 ou C6,
* un radical acyle, linéaire ou ramifié, saturé ou insaturé, en C2-C18, ou cyclique saturé ou insaturé en C5 ou C6,
**Rₐ** désigne :
- un atome d'hydrogène
- un radical alkyle linéaire ou ramifié, en C1-C4, ou un radical hydrocarboné insaturé linéaire ou ramifié en C3-C4
- un radical acyle linéaire ou ramifié en C2-C18 ou alcénylcarbonyle linéaire ou ramifié en C2-C18
**R_{b}** désigne :
- un atome d'hydrogène
- un radical alkyle linéaire en C2-C18, ou ramifié en C3-C18, ou un radical hydrocarboné insaturé linéaire ou ramifié en C3-C4 ou un radical -CH(Z₁₎-CO₂Z₂ dans lequel
Z₁ désigne un atome d'hydrogène ou un radical alkyle linéaire ou ramifié en C1-C6, ou cyclique saturé ou insaturé en C3-C6, ledit radical étant éventuellement substitué par au moins un groupement choisi parmi =NH, -NH₂, -N(T)₂, =O, -OH, -OT, -SH, -ST, -CO₂T, phényle, phényle substitué par -OH ou -OT, 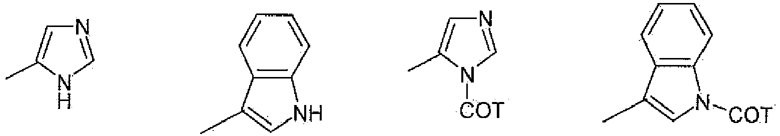 et/ou interrompu par un groupement -NH-, -N-(COT)-, ou -S- avec T désignant un radical alkyle linéaire ou ramifié en C1-C6, ou cyclique en C3-C6.
et Z₂ désigne un atome d'hydrogène ou un radical alkyle linéaire ou ramifié en C1-C6
**R_{c}** désigne :
- un atome d'hydrogène
- un radical alkyle linéaire en C1-C4, ou ramifié en C3-C4, ou un radical hydrocarboné insaturé linéaire ou ramifié en C3-C4, ledit radical étant éventuellement substitué par un groupement phényle.
Rd désigne :
- un atome d'hydrogène
- un radical alkyle linéaire en C1-C18, ou ramifié en C3-C18, ou un radical hydrocarboné insaturé linéaire ou ramifié en C3-C18, ledit radical étant éventuellement substitué par un groupe phényle,
la liaison S-CH₂-X représente une liaison de nature C-anomérique, qui peut être α ou β,
ainsi que leurs sels cosmétiquement acceptables, leurs solvates tels que les hydrates et leurs isomères optiques et géométriques.

11. Composition cosmétique selon l'une quelconque des revendications 1 à 10, dans laquelle le composé C-glycoside (c) est représenté par la formule suivante : 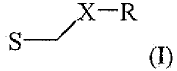 dans laquelle :
- R désigne un radical alkyle linéaire non substitué en C₁-C₄, notamment
C₁-C₂, en particulier méthyle ;
- S représente un monosaccharide comme décrit précédemment et en particulier choisi parmi le D-glucose, le D-xylose, la N-acétyl-D-glucosamine ou le L-fucose, et en particulier le D-xylose ;
- X représente un groupement choisi parmi -CO-, -CH(OH)-, -CH(NH₂)-, et préférentiellement un groupement -CH(OH)- .

12. Composition cosmétique selon l'une quelconque des revendications 1 à 11, dans laquelle le composé C-glycoside (c) est le C-β-D-xylopyranoside-2-hydroxy-propane ou le
C-α-D-xylopyranoside-2-hydroxy-propane, et de préférence le
C-β-D-xylopyranoside-2-hydroxy-propane.

13. Composition cosmétique selon l'une quelconque des revendications 1 à 10, dans laquelle le composé C-glycoside (c) est représenté par la formule (II) suivante : 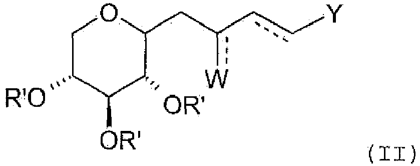 dans laquelle,
- les composés de formule (II) sont des dérivés de xylose
- Y désigne un radical phényle ou un hétérocycle, éventuellement substitué par 1 à 5 groupements (ORₐ)
- W = -OR' ; (=O) ; NR_{b}R_{c} ; NHOR_{d}
- R' désigne :
* un atome d'hydrogène,
* un radical alkyle linéaire et saturé en C1-C18,
* un radical alkyle linéaire et insaturé C2-C18,
* un radical alkyle ramifié, saturé ou insaturé, en C3-C18,
* un radical cyclique saturé ou insaturé en C5 ou C6,
* un radical acyle, linéaire ou ramifié, saturé ou insaturé, en C2-C18, ou cyclique saturé ou insaturé en C5 ou C6.
**Rₐ** désigne :
- un atome d'hydrogène
- un radical alkyle linéaire ou ramifié en C1-C4, ou un radical hydrocarboné insaturé linéaire ou ramifié en C3-C4
- un radical acyle linéaire ou ramifié en C2-C18 ou un radical alcénylcarbonyle linéaire ou ramifié en C2-C18
- lorsque Y désigne un radical phényle ou un hétérocycle substitué par 2 à 5 groupements (ORₐ), deux groupements adjacents ORₐ peuvent former ensemble un radical divalent -O-CH₂-O
sous réserve que lorsque W=OH, le composé ne comporte pas de double liaison éthylénique en position alpha du carbone portant cet OH.
**R_{b}** désigne :
- un atome d'hydrogène
- un radical alkyle linéaire en C2-C18, ou ramifié en C3-C18, ou un radical hydrocarboné insaturé linéaire ou ramifié en C3-C4 ou un radical -CH(Z₁₎-CO₂Z₂ dans lequel
Z₁ désigne un atome d'hydrogène ou un radical alkyle linéaire ou ramifié en C1-C6, ou cyclique saturé ou insaturé en C3-C6, ledit radical étant éventuellement substitué par au moins un groupement choisi parmi =NH, -NH₂, -N(T)₂, =O, -OH, -OT, -SH, -ST, -CO₂T, phényle, phényle substitué par -OH ou -OT, 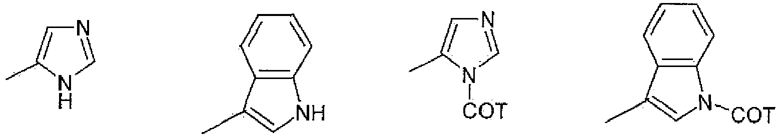 et/ou interrompu par un groupement -NH-, -N-(COT)-, ou -S- avec T désignant un radical alkyle linéaire ou ramifié en C1-C6, ou cyclique en C3-C6.
et Z₂ désigne un atome d'hydrogène ou un radical alkyle linéaire en C1-C6
**R_{c}** désigne :
- un atome d'hydrogène
- un radical alkyle linéaire en C1-C4, ou ramifié en C3-C4, ou un radical hydrocarboné insaturé linéaire ou ramifié en C3-C4, ledit radical étant éventuellement substitué par un groupement phényle.
**Rd** désigne :
- un atome d'hydrogène
- un radical alkyle linéaire en C1-C18, ou ramifié en C3-C18, ou un radical hydrocarboné insaturé linéaire ou ramifié en C3-C18, ledit radical étant éventuellement substitué par un groupe phényle,
ainsi que leurs sels cosmétiquement acceptables, leurs solvates tels que les hydrates et leurs stéréoisomères.

14. Composition selon l'une quelconque des revendications 1 à 10 ou 13, **caractérisée par le fait que** le composé C-glycoside (c) est de formule (II) dans laquelle :
- les composés de formule (II) sont des dérivés de xylose
- Y désigne un radical phényle ou un hétérocycle, éventuellement substitué par 1 à 3 groupements (ORₐ)
- W = -OR' ; (=O) ; NR_{b}R_{c} ; NHOR_{d}
- R' désigne :
* un atome d'hydrogène,
* un radical alkyle linéaire ou ramifié, saturé ou insaturé en C1-C4,
* un radical acyle linéaire ou ramifié en C1-C6,
**Rₐ** désigne :
- un atome d'hydrogène
- un radical alkyle linéaire ou ramifié, en C1-C4,
- un radical acyle linéaire ou ramifié en C1-C6, lorsque Y désigne un radical phényle ou un hétérocycle substitué par 2 ou 3 groupements (ORₐ), deux groupements adjacents ORₐ peuvent former ensemble un radical divalent -O-CH₂-O
sous réserve que lorsque W=OH, le composé ne comporte pas de double liaison éthylénique en position alpha du carbone portant cet OH
**R_{b}** désigne :
- un atome d'hydrogène
- un radical alkyle linéaire en C2-C8, ou ramifié en C3-C8, ou un radical -CH (Z₁₎-CO₂Z₂ dans lequel Z₁ désigne un atome d'hydrogène ou un radical alkyle linéaire ou ramifié en C1-C6, ou cyclique saturé ou insaturé en C3-C6, ledit radical étant éventuellement substitué par au moins un groupement choisi parmi =NH, -NH₂, -N(T)₂, =O, -OH, -OT, -SH, -ST, -CO₂T, phényle, phényle substitué par -OH ou -OT, 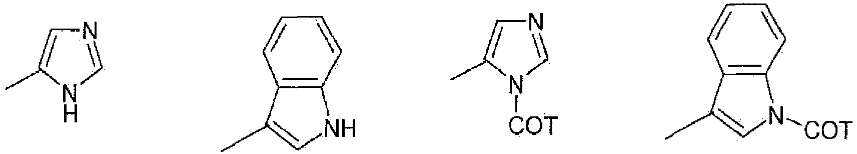 et/ou interrompu par un groupement -NH-, -N-(COT)-, ou -S- avec T désignant un radical alkyle linéaire en C1-C6, cyclique en C5-C6.
et Z₂ désigne un atome d'hydrogène ou un radical alkyle linéaire ou ramifié en C1-C6
**R_{c}** désigne :
- un atome d'hydrogène
- un radical alkyle linéaire en C1-C4, ou ramifié en C3-C4, éventuellement substitué par un groupement phényle,
**Rd** désigne :
- un atome d'hydrogène
- un radical alkyle linéaire en C1-C4, ou ramifié en C3-C4, ledit radical étant éventuellement substitué par un groupe phényle,
ainsi que leurs sels cosmétiquement acceptables, leurs solvates tels que les hydrates et leurs stéréoisomères.

15. Composition selon l'une quelconque des revendications 1 à 10 ou 13, **caractérisée par le fait que** le composé C-glycoside (c) est de formule (II) et est choisi parmi :
**Composé 1.** : (3R,4S,5R)-2-[2-hydroxy-4-(4-hydroxy-3-méthoxyphényl)butyl]tétrahydro-2H-pyran-3,4,5-triol 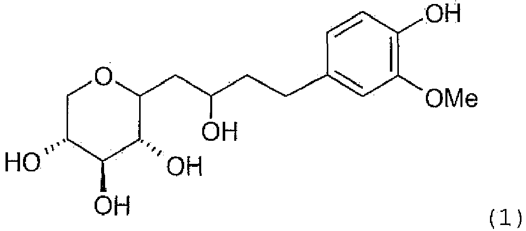
**Composé 2.** 4-(4-hydroxy-3-méthoxyphényl)-1-[(3R,4S,5R)-3,4,5-trihydroxytétrahydro-2H-pyran-2-yl]butan-2-one 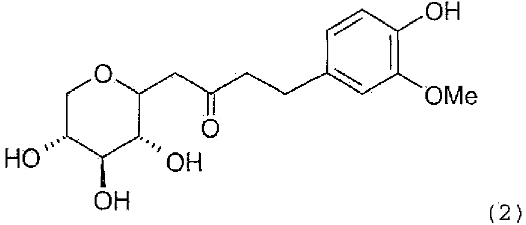
**Composé 3.** (3R,4S,5R)-2-[2-hydroxy4-(4-hydroxyphényl)butyl]tétrahydro-2H-pyran-3,4,5-triol 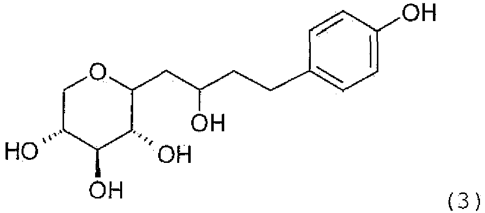
**Composé 4** 4-(4-hydroxy-phényl)-1-[(3R,4S,5R)-3,4,5-trihydroxytétrahydro-2H-pyran-2-yl]butan-2-one 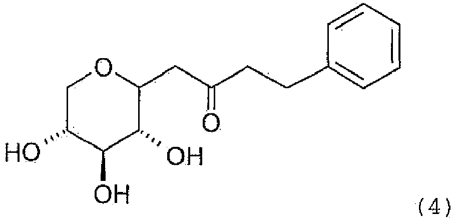
**Composé 5.** (3R,4S,5R)-2-[2-(benzylamino)-4-(4-hydroxy-3-méthoxyphényl)butyl]tétrahydro-2H-pyran-3,4,5-triol 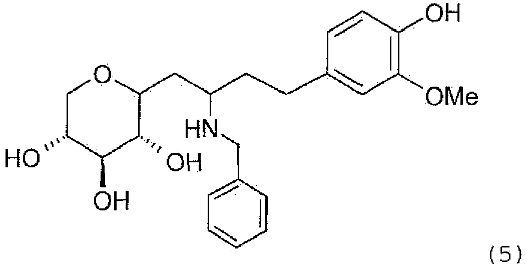
**Composé 6.** éthyl {[3-(4-hydroxy-3-méthoxyphényl)-1-{[(3R,4S,5R)-3,4,5-trihydroxytétrahydro-2H-pyran-2-yl]methyl}propyl]amino}(phényl)acétate 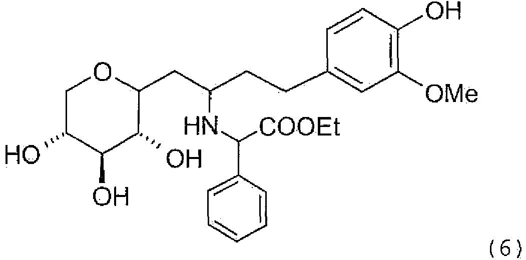
**Composé 7.** (3E)-4-phényl-1-[(3R,4S,5R)-3,4,5-trihydroxytétrahydro-2H-pyran-2-yl]but-3-en-2-one 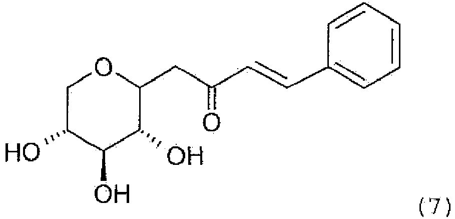
**Composé 8.** (3E)-4-(4-hydroxy-3,5-diméthoxyphényl)-1-[(3R,4S,5R)-3,4,5-trihydroxytétrahydro-2H-pyran-2-yl]but-3-en-2-one 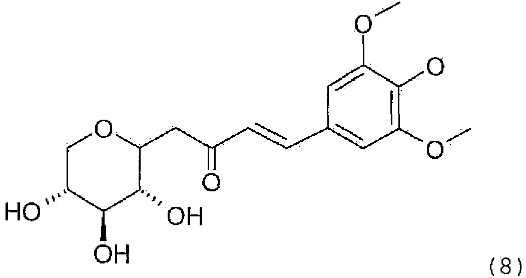
**Composé 9.** (3R,4S,5R)-2-[2-hydroxy-4-(2-hydroxyphényl)butyl]tétrahydro-2H-pyran-3,4,5-triol 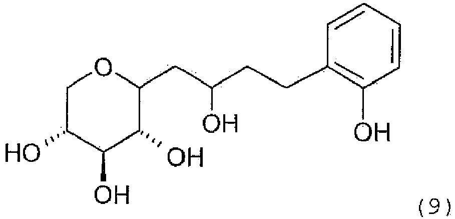
**Composé 10 :** (3R,4S,5R)-2-[2-hydroxy-4-(3-hydroxy-4-méthoxyphényl)butyl]tétrahydro-2H-pyran-3,4,5-triol 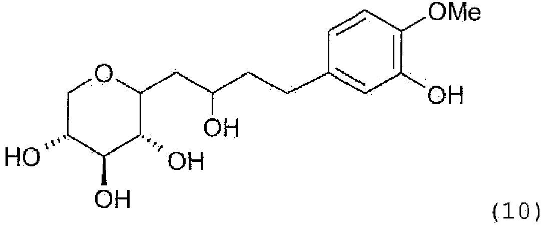
**Composé 11.** (3R,4S,5R)-2-[2-hydroxy-4-(2,4-dihydroxyphényl)butyl]tétrahydro-2H-pyran-3,4,5-triol 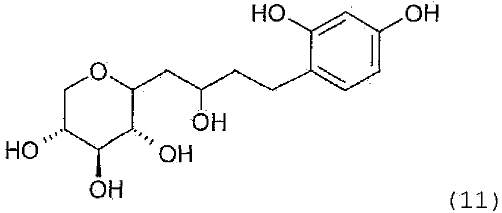
**Composé** 12. (3R,4S,5R)-2-[2-hydroxy-4-(3-ethoxy-4-hydroxyphényl)butyl]tétrahydro-2H-pyran-3,4,5-triol 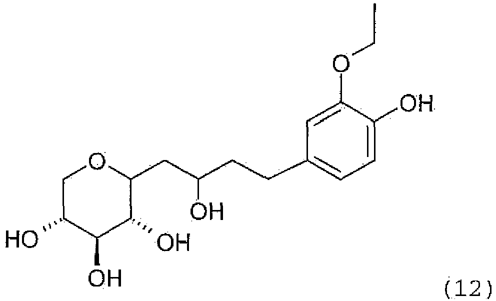
**Composé** 13. 5,9-anhydro-1,2,4-tridésoxy-1-pyridin-3-yl-D-xylononitol 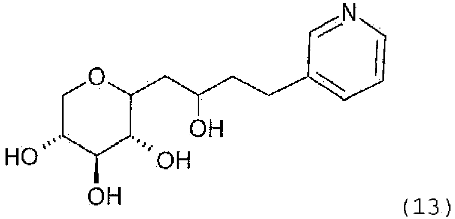
**Composé 14.** (3R,4S,5R)-2-[(2E)-2-(méthoxyimino)-4-phénylbutyl]tétrahydro-2H-pyran-3,4,5-triol 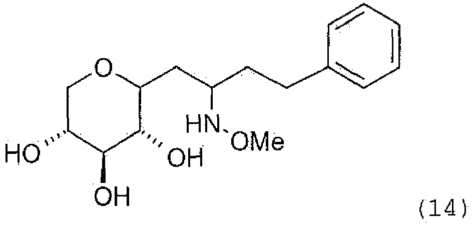
**Composé 15.** 5,9-anhydro-1,2,4-tridésoxy-7-O-pentanoyl-1-phényl-D-xylonon-3-ulose 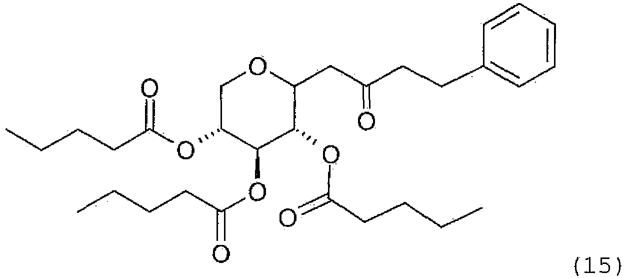
**Composé 16.** 5,9-anhydro-1,2,4-tridésoxy-1-(3,4,5-triméthoxyphényl)-D-xylononitol

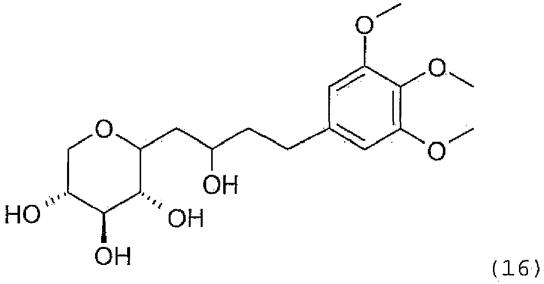

16. Composition selon la revendication précédente, **caractérisée par le fait que** le composé C-glycoside (c) est de formule (II) et est choisi parmi les composés (1) et (11).

17. Composition cosmétique selon l'une quelconque des revendications 1 à 16, dans laquelle la quantité du composé C-glycoside (c) est dans la plage de 0,01 à 20 % en poids, de préférence de 0,1 à 15 % en poids, et plus préférablement de 1 à 10 % en poids, par rapport au poids total de la composition.

18. Composition cosmétique selon l'une quelconque des revendications 1 à 17, comprenant en outre au moins un tensioactif non ionique différent de (b) ci-dessus et/ou au moins un tensioactif ionique.

19. Composition cosmétique selon l'une quelconque des revendications 1 à 18, comprenant en outre au moins un polyol.

20. Composition cosmétique selon l'une quelconque des revendications 1 à 19, dans laquelle la composition cosmétique est sous la forme d'une émulsion H/E, et l'huile (a) est sous la forme de gouttelettes ayant une taille de particule moyenne en nombre de 300 nm ou moins, de préférence de 10 nm à 150 nm.

21. Procédé non thérapeutique pour traiter la peau, les cheveux, les membranes muqueuses, les ongles, les cils, les paupières et/ou le cuir chevelu, **caractérisé en ce que** la composition cosmétique selon l'une quelconque des revendications 1 à 20 est appliquée sur la peau, les cheveux, les membranes muqueuses, les ongles, les cils, les paupières ou le cuir chevelu.

22. Utilisation de la composition cosmétique selon l'une quelconque des revendications 1 à 20, en tant que ou dans des produits de soin et/ou des produits de lavage et/ou des produits de maquillage et/ou des produits démaquillants pour la peau du corps et/ou du visage et/ou les membranes muqueuses et/ou le cuir chevelu et/ou les cheveux et/ou les ongles et/ou les cils et/ou les paupières.
